# EUROPEAN PATENT APPLICATION

(11) **EP 3 742 173 A1**
(43) Date of publication of application: **25.11.2020**
(21) Application number: 19740986.5
(22) Date of filing: 18.01.2019
(51) Int. Cl.: G01N 35/04, G01N 33/48, G01N 37/00

(54) **FLOW PATH, MEASUREMENT TAPE, AND MEASURING DEVICE**

(30) Priority: 19.01.2018 JP 2018007385
(71) Applicant: NITTO DENKO CORPORATION, Ibaraki-shi Osaka 567-8680 (JP)
(72) Inventor: MIWA, Kazuya, Ibaraki-shi, Osaka 567-8680 (JP); MASUDA, Ryota, Ibaraki-shi, Osaka 567-8680 (JP); MIYAHARA, Makoto, Ibaraki-shi, Osaka 567-8680 (JP); TSUJI, Takashi, Ibaraki-shi, Osaka 567-8680 (JP); HORI, Mitsuhiko, Ibaraki-shi, Osaka 567-8680 (JP)
(74) Representative: Grünecker Patent- und Rechtsanwälte PartG mbB
(86) International application number: PCT/JP2019/001560
(87) International publication number: WO 2019/142928

(57) **Abstract**

Provided is a flow path that can easily feed a liquid in the flow path.

A flow path (210) is the flow path (210) for feeding a liquid, at least a portion of the flow path (210) includes a first region having a plurality of first through-holes for maintaining a pressure in the flow path (210) at atmospheric pressure, and a second region having an affinity for the liquid, and the liquid is fed by a force caused by affinity for the second region.

## Description

### Technical Field

The present invention relates to a flow path for feeding a liquid, a measurement tape provided with a flow path, and a measurement device that performs measurement using the measurement tape.

### Background Art

In the related art, there is known a flow cell in which a sample solution is caused to flow in a hollow chamber having a square cross section of a flow path, and a scattered light emitted from a sample is detected by irradiating the sample solution with a collected laser light. As one of the flow cells, a flow cell including a sample non-affinity substrate and a flow path of a porous member provided on the sample non-affinity substrate is known. In this flow cell, the porous member includes an outside air non-contact region having a mesh structure and a sample affinity, and an outside air contact region that covers the outside air non-contact region and has a lower pore density than that of the outside air non-contact region. A capillary force generated in the porous member serves as a driving force for liquid feeding (refer to Patent Document 1).

### Related Art

### Patent Document

[Patent Document 1] International Publication No. WO2008/001737

### Disclosure of Invention

### Problems to Be Solved by the Invention

In a flow path of Patent Document 1, a driving force for liquid feeding is a capillary force generated in a porous member. Thus, the absence of the porous member in the flow path leads to the absence of the driving force for liquid feeding and the liquid feeding is difficult. In addition, particles of the sample easily enter the pores of the porous member in the flow path. For example, when the viscosity of the sample particles is high, the sample particles are particularly likely to block the porous pores, and this makes the feeding of the sample difficult. In addition, when the sample is a protein biomaterial, the sample is easily adsorbed on the porous member to easily block the porous pores. This makes the feeding of the sample difficult.

The present invention has been made in view of the above circumstances, and provides a flow path, a measurement tape, and a measurement device that can easily feed a liquid in a flow path.

### Means for Solving the Problems

One embodiment of the present invention is a flow path for feeding a liquid, wherein at least a portion of the flow path comprises: a first region having a plurality of first through-holes for maintaining a pressure in the flow path at atmospheric pressure; and a second region having an affinity for the liquid, and wherein the liquid is fed by a force caused by the affinity for the second region.

One embodiment of the present invention is a measurement tape comprising the above flow path, wherein the first region and the second region which are included in the flow path have flexibility to be bendable in an arrangement direction where the first region and the second region are arranged.

One embodiment of the present invention is a measurement device which performs measurement using the above measurement tape, the device comprising: the measurement tape including the flow path; a light source; a light receiving unit; and a measurement unit, wherein the flow path includes a first space defined at one end of the flow path and a second space defined at the other end of the flow path, the liquid is introduced into the first space, and is fed from the first space to the second space, the light source emits measurement light to the second space, the light receiving unit receives scattered light in which the measurement light is scattered in the second space, and the measurement unit measures a state of the liquid fed through the flow path based on the scattered light.

### Effects of the Invention

According to this invention, the liquid in a flow path can be easily fed.

### Brief Description of Drawings

FIG. 1 is a diagram illustrating a schematic configuration of a drain effluent management system according to an embodiment.
FIG. 2A is a diagram illustrating an internal configuration of a drain effluent sensor.
FIG. 2B is a block diagram illustrating a circuit configuration of a sensor unit.
FIG. 3 is a view illustrating a shape of a drain bag.
FIG. 4 is a partially cutaway perspective view illustrating an appearance of a drain effluent monitor.
FIG. 5 is a diagram describing an operation of an effluent sampling mechanism.
FIG. 6 is a flowchart illustrating a procedure of an effluent sampling operation.
FIG. 7A is an exploded perspective view illustrating an example of a flow path formed in a measurement tape 200.
FIG. 7B is a plan view illustrating an example of an adhesive layer in the flow path.
FIG. 7C is a cross-sectional view illustrating an example of the flow path.
FIG. 8A is a cross-sectional view illustrating an example of introduction of a sampling liquid into the flow path.
FIG. 8B is a cross-sectional view illustrating an example of a flow of the sampling liquid separated by a blood cell separation membrane in the flow path.
FIG. 8C is a cross-sectional view illustrating an example in which sampling reached a space at a liquid feeding destination in a flow path.
FIG. 9 is a flowchart illustrating a procedure for measuring amylase activity.
FIG. 10 is a diagram illustrating a display on a drain effluent monitor.
FIG. 11 is a diagram describing introduction of a biological fluid containing a drug or the like into a patient by a TCI pump.

### Embodiments for Carrying Out the Invention

Hereinafter, embodiments of a flow path, a measurement tape, and a measurement device according to the present invention will be described with reference to the drawings. In the present embodiment, a drain effluent management system is illustrated as an example of a management system of a drain effluent. The drain effluent management system measures a state of a component to be managed contained in a drain effluent flowing through a drain tube for drainage connected to a patient's body, and performs various observations and evaluations on the state of the drain effluent.

The drain effluent is a liquid that is drained out of the body after surgery and contains blood, exudate and the like accumulated in the body. When a digestive system operation is performed, amylase, which is a digestive enzyme, and bilirubin, which is a bile pigment, may come out from the affected area. These components damage organs and dissolve blood vessels to be likely to occur bleeding and thus may be a risk of complications. Therefore, measuring the state of the component to be managed contained in the drain effluent is a great index for medical staff to consider a next medical practice. For example, it is conceivable to measure the state of amylase, bilirubin, and blood as the component to be managed in the drain effluent.

### (Configuration of Drain Effluent Management System)

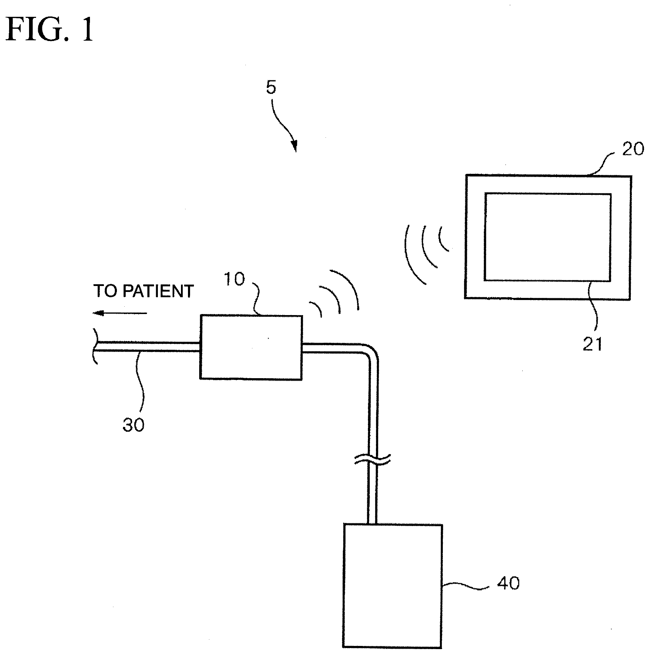

FIG. 1 is a diagram illustrating a schematic configuration of a drain effluent management system 5 according to the embodiment. The drain effluent management system 5 is provided with a non-contact type drain effluent sensor 10, a drain effluent monitor 20, a drain tube 30, and a drain bag 40. The drain effluent sensor 10 is used for measuring the state of the components to be managed (for example, blood, amylase, and bilirubin), which are the components to be managed in the drain effluent flowing through a transparent drain tube 30. That is, the drain effluent sensor 10 is an example of a measurement device. As the component to be managed, amylase is a digestive enzyme secreted from the pancreas and salivary glands. Bilirubin is a yellow pigment component contained in bile. Blood is bleeding from organs and blood vessels.

The drain effluent monitor 20 is connected to the drain effluent sensor 10 by wireless communication or wired communication, and records and displays a measurement result by the drain effluent sensor 10. A display 21 for displaying various information is disposed on a front surface of the drain effluent monitor 20.

The drain tube 30 lets the drain effluent from the patient's body flow through and let it flow into the drain bag 40. The drain tube 30 is formed of a material such as resin having a light-transmitting property so that a user can visually observe the drain effluent. One end of the drain tube 30 is connected (inserted) to the patient's body, and the other end of the drain tube 30 is connected to the drain bag 40. The drain bag 40 stores the drain effluent flowing from the drain tube 30.

The drain effluent stored in the drain bag 40 includes a discharged material flowed from the start of drainage to the time of measurement. Therefore, it is difficult to measure the state of the component to be managed in the drain effluent, which changes over time, in association with time, using the drain effluent.

Therefore, in the drain effluent management system 5, the state of the component to be managed in the drain effluent is measured using the drain effluent flowing through the drain tube 30 connected to the patient's body and the drain bag 40. This method allows the drain effluent management system 5 to perform measurement on the patient non-invasively.

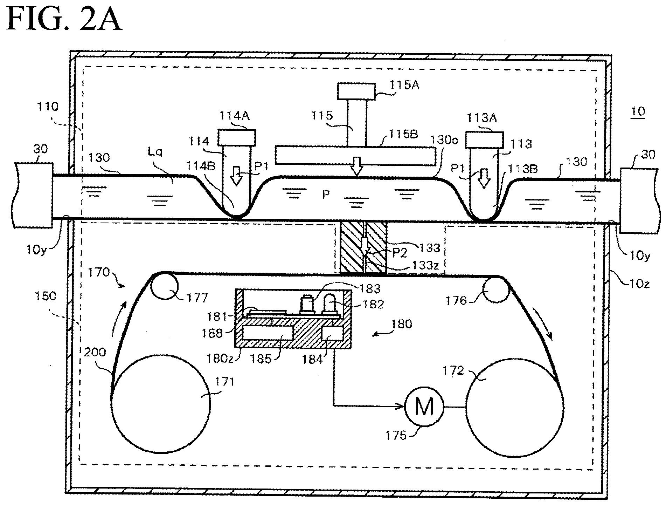

FIG. 2A is a diagram illustrating an internal configuration of the drain effluent sensor 10. The drain effluent sensor 10 has, for example, a box-shaped case 10z, and accommodates an effluent sampling mechanism 110 and a blood cell separation and enzyme reaction mechanism 150 inside the case 10z.

In the effluent sampling mechanism 110, a main tube 130 (an example of main flow path) is attached so as to penetrate the inside of the case 10z. In FIG. 2A, the main tube 130 is provided in a left-right direction. Both ends of the main tube 130 may project from through-holes 10y formed on both side surfaces of the case 10z, and may be connected to both ends of the drain tube 30. The main tube 130 may be a portion of the drain tube 30.

A sub-tube 133 (an example of a sub-flow path) branched from the main tube 130 is connected to substantially a center of the main tube 130. Here, a position of the main tube 130 to which one end of the sub-tube 133 is connected is also referred to as a branch point. The sub-tube 133 has an elongated flow path 133z that can communicate with the inside of the main tube 130. The branch point may be a connection position between the sub-tube 133 and the flow path 133z. The sub-tube 133 may be formed of a material having elasticity (rubber, resin, or the like) and may be a material that is elastically recovered. The sub-tube 133 is deformed within a range where it is elastically deformed, and is not deformed within a range where it is plastically deformed. The elongated flow path 133z is normally closed. Since the flow path 133z is normally closed, a drain effluent Lq in the main tube 130 does not flow into the flow path 133z of the sub-tube 133, and the liquid in the flow path 133z of the sub-tube 133 does not flow back to the main tube 130. In addition, gas does not flow in from the side of the sub-tube 133 opposite to the side of the main tube 130 (lower side in FIG. 2A).

When viewed from the main tube 130, the sub-tube 133 can be said to be a protruding portion formed to protrude in the middle of the main tube 130. It can be said that a cut is formed as the flow path 133z in this protruding portion.

The effluent sampling mechanism 110 includes a pair of restriction members 113 and 114 and a first pressing member 115, in addition to the main tube 130 and the sub-tube 133.

Each of the pair of restriction members 113 and 114 includes partition plates 113B and 114B formed with curved distal ends, and pressurizing units 113A and 114A. The partition plates 113B and 114B driven by the pressurizing units 113A and 114A move so as to press each portions on both sides (for example, two points) interposing the branch point of the main tube 130. The pair of restriction members 113 and 114 can press the portions on both sides of the main tube 130 with the branch point of the sub-tube 133 interposed therebetween. When the pair of restriction members 113 and 114 press the portions on both sides of the main tube 130 substantially simultaneously, the drain effluent Lq is restricted by the main tube 130 and, for example, does not flow. Therefore, the drain effluent Lq stays in a tube central portion 130c of the main tube 130 located in the vicinity of the branch point. The tube central portion 130c may be, for example, a region between two points pressed by the restriction members 113 and 114.

The first pressing member 115 includes a pressing plate 115B having a flat plate which is flush with the main tube 130 along the longitudinal direction, and a pressurizing unit 115A. The pressing plate 115B driven by the pressurizing unit 115A moves so as to press the main tube 130. When the first pressing member 115 presses the main tube 130, the pressure of the drain effluent Lq staying in the tube central portion 130c of the main tube 130 closed by the pair of restriction members 113 and 114 increases.

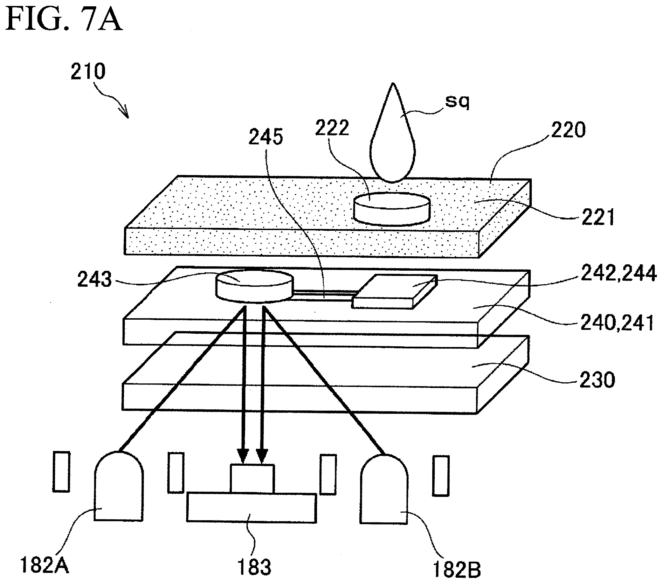

When the pressure of the drain effluent Lq in the tube central portion 130c increases, the flow path 133z of the sub-tube 133 opens, and the drain effluent Lq staying in the tube central portion 130c flows into the flow path 133z of the sub-tube 133. The effluent having flowed into the flow path 133z of the sub-tube 133 flows out so as to be pushed out from the end surface on a side opposite to the flow path 133z. The drain effluent Lq having flowed out from the end surface on the side opposite to the flow path 133z is dropped as a sampling liquid sq (refer to FIG. 7A) onto a flow path 210 of a measurement tape 200 disposed so as to face the sub-tube 133.

The pressurizing units 113A, 114A, and 115A (described later) move the partition plates 113B and 114B, and the pressing plate 115B in the forward and rearward directions, respectively, according to the drive signal from a sensor unit 180. For example, when the pressurizing unit is configured to include a motor gear mechanism, each of the partition plates 113B and 114B, and the pressing plate 115B move straight by driving the motor. The pressurizing unit is not limited to the motor gear mechanism, and may be configured to include an electromagnetic slide mechanism, a piezoelectric element, a hydraulic slide mechanism, or the like.

The blood cell separation and enzyme reaction mechanism 150 separates the blood cells contained in the drain effluent Lq, causes the enzyme to react with the reagent, and optically measures the absorbance of the enzyme. The blood cell separation and enzyme reaction mechanism 150 may optically measure the absorbance of a component to be managed other than the enzyme (for example, blood cells, bilirubin). The blood cell separation and enzyme reaction mechanism 150 has a tape winding and feeding mechanism 170 and a sensor unit 180.

The tape winding and feeding mechanism 170 includes a measurement tape 200 onto which the sampling liquid sq is dropped, a feed reel 171 around which the unused measurement tape 200 is wound, and a winding reel 172 around which the reacted measurement tape 200 is wound. In addition, the tape winding and feeding mechanism 170 includes a motor 175 for driving the winding reel 172, and rollers 177 and 176 for guiding the movement of the measurement tape 200. The winding reel 172 is rotated by the drive of the motor 175 and winds the measurement tape 200 after the reaction. The feed reel 171 rotates with the movement of the measurement tape 200.

Here, the winding reel 172 rotates so as to wind the measurement tape. Each of the winding reel 172 and the feed reel 171 may be driven by a motor to simultaneously wind and feed the measurement tape 200, so that the movement of the measurement tape 200 can be further stabilized. In addition, only the feed reel 171 may be driven by a motor and the winding reel 172 may rotate.

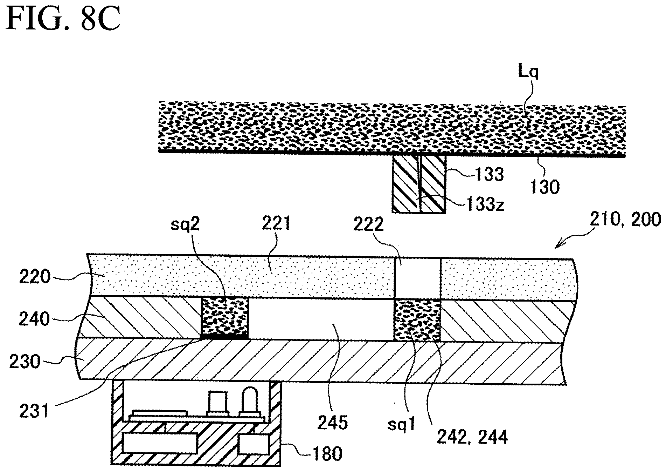

The sensor unit 180 is disposed so as to face the effluent sampling mechanism 110 with the measurement tape 200 interposed therebetween. The sensor unit 180 optically measures the absorbance of the enzyme contained in the sampling liquid sq2 (refer to FIG. 8C) permeated through the measurement tape 200 sent out by the tape winding and feeding mechanism 170. In this measurement, the sensor unit 180 projects light having a peak value at a predetermined wavelength (for example, wavelength of 410 nm) as measurement light toward the measurement tape 200 into which the sampling liquid sq2 containing a large amount of enzyme is permeated. The sensor unit 180 may receive the scattered light that is not absorbed by the enzyme in the sampling liquid sq2 and measure the absorbance of the enzyme contained in the sampling liquid sq2 based on the amount of scattered light received.

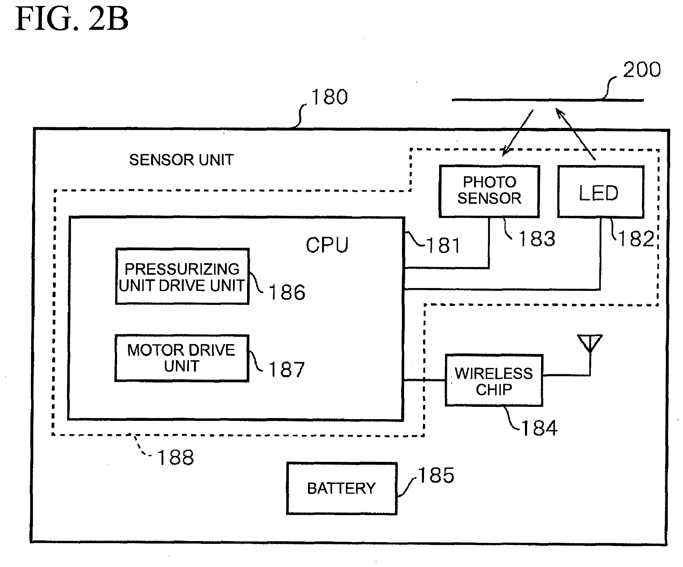

FIG. 2B is a block diagram illustrating a circuit configuration of the sensor unit 180. The sensor unit 180 includes a case 180z in which a circuit substrate 188 is laid. The sensor unit 180 includes a central processing unit (CPU) 181, a light emitting diode (LED) 182, a photo sensor (PD) 183, a wireless chip 184, and a battery 185. On the circuit substrate 188, the CPU 181, the photo sensor 183, and the LED 182 are mounted.

The CPU 181 controls the operation of each unit in the sensor unit 180. The CPU 181 may perform various arithmetic processes such as calculating absorbance based on the amount of light received from the photo sensor 183. The CPU 181 includes functions as a pressurizing unit drive unit 186 and a motor drive unit 187. The pressurizing unit drive unit 186 outputs a drive signal to each of the pressurizing units 113A, 114A, and 115A. The motor drive unit 187 outputs a drive signal to the motor 175 that rotates the winding reel 172. The CPU 181 has a timing function, and may measure the time at the time of sampling or the time at the time of measurement.

The CPU 181 generates measurement data such as absorbance. The measurement data is data relating to a component to be managed (for example, blood, amylase, and bilirubin). The measurement data may be managed in association with the time at which the measurement data is measured. The measurement data may include, for example, information on the absorbance of the component to be managed and the amount of change in absorbance. The measurement data may include information on a discharge amount of the component to be managed per unit time. The measurement data may include information on a total discharge amount of the component to be managed. The discharge amount of the component to be managed may be a discharge amount of the component to be managed flowing through the main tube 130, or a discharge amount (sampling amount) of the component to be managed sampled via the sub-tube 133.

The LED 182 emits light having a peak value at a predetermined wavelength (for example, wavelength of 410 nm) as measurement light. The photo sensor 183 receives scattered light without being absorbed by the component to be managed such as amylase, and outputs a signal corresponding to the amount of light received.

In addition, the LED 182 may be an LED that can emit visible light or an LED that can emit visible light and invisible light (for example, infrared light or ultraviolet light). The LED 182 may include a plurality of LEDs. For example, the LED 182 may include an LED 182A that emits visible light and an LED 182B that emits infrared light (refer to FIG. 7A).

The wireless chip 184 performs wireless communication with the drain effluent monitor 20, and transmits various data measured by the sensor unit 180 to the drain effluent monitor 20. For wireless communication, communication such as short-range wireless communication (such as Bluetooth (registered trademark) and ZigBee (registered trademark)) or wireless local area network (LAN) can be used. The battery 185 supplies power to each unit of the sensor unit 180. The battery 185 may be a secondary battery such as a rechargeable lithium-ion battery or a primary battery such as an alkaline battery.

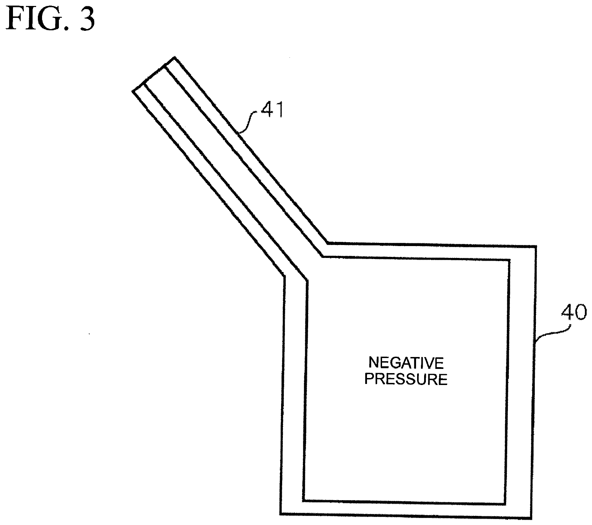

FIG. 3 is a view illustrating a shape of the drain bag 40. The drain bag 40 is a bag for storing the drain effluent. An inflow tube 41 through which the drain effluent flows is attached to the drain bag 40. A distal end of the inflow tube 41 is connected to one end of the drain tube 30. In addition, the inflow tube 41 may be a portion of the drain tube 30. The drain effluent drained from the drain tube 30 flows into the drain bag 40 and is stored. In addition, since the inside of the drain bag 40 is maintained at a negative pressure, even if an distal end of the inflow tube 41 is connected to one end of the drain tube 30, the drain effluent stored in the drain bag 40 does not flow back to the drain tube 30. In addition, by maintaining the negative pressure, infection of a patient who discharges the drain effluent can be prevented.

Here, the main tube 130 is connected to the drain tube 30, and the sub-tube 133 branches from the main tube 130. The flow path 133z is formed in the sub-tube 133 to sample and extract the drain effluent Lq. However, when the drain effluent Lq does not pass through the sub-tube 133, the flow path 133z is basically closed. Therefore, it is possible for the sub-tube 133 to suppress the entry of air and the like to the main tube 130 side (drain tube 30 side) from the flow path 133z. Therefore, even when the drain effluent sensor 10 is provided with the main tube 130 and the sub-tube 133, it is possible to suppress the entry of air and the like from the sub-tube 133 and maintain the negative pressure of the drain bag 40. Therefore, it is possible to suppress the drain effluent stored in the drain bag 40 from flowing back to the drain tube 30. Even after one week in a state where the drain effluent sensor 10, the drain tube 30, and the drain bag 40 are connected to each other, it was confirmed that gas such as air was not substantially included in the drain bag 40.

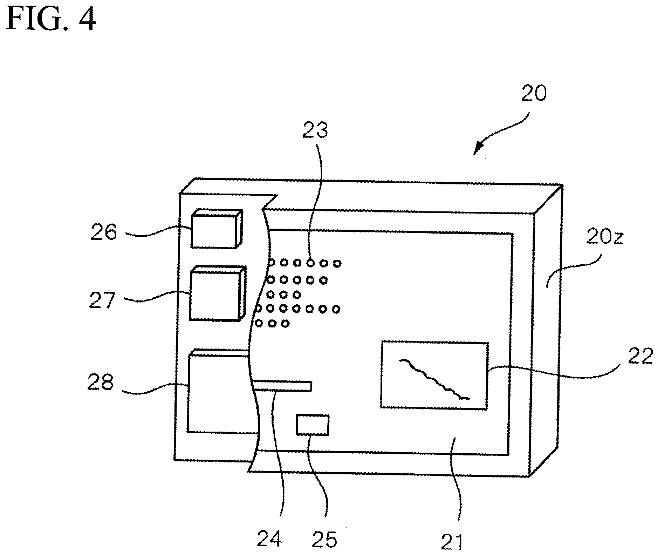

FIG. 4 is a partially cutaway perspective view illustrating an appearance of the drain effluent monitor 20. The drain effluent monitor 20 includes a box-shaped case 20z. The display 21 is disposed on the front surface of the case 20z. The display 21 displays a graph 22 representing a measurement result over time, various explanatory texts 23 such as a patient's name, a meter 24 representing a current value of the measurement result, a status marker 25 for notifying a patient's state (normal or abnormal) and the like.

The drain effluent monitor 20 is provided with a CPU 26 for performing various controls of the drain effluent monitor 20, a memory 27 for recording measurement data of the drain effluent sensor 10, and the wireless chip 28 for performing wireless communication with the wireless chip 184 of the drain effluent sensor 10.

In addition, an internal clock (not illustrated) is built in the drain effluent monitor 20, and the measurement data measured by the drain effluent sensor 10 (specifically, sensor unit 180) and the time when the measurement data is measured are recorded in the memory 27 in association with each other. The measurement data may include information on a discharge amount of the component to be managed per unit time. The information on the discharge amount of the drain effluent Lq of the component to be managed per unit time may include, for example, information on the absorbance of the component to be managed (for example, amylase, bilirubin, and blood) and the amount of change in absorbance per unit time. The graph 22 is displayed based on the time-series data recorded in the memory 27 so as to represent a change over time of the measurement result. In addition, the measurement data may be information on the total discharge amount of the component to be managed.

The CPU 26 may register (hold) a portion or all of the time-series data recorded in the memory 27 and at least one of the display results of the graph 22 in an electronic medical record for each patient. The electronic medical record may be stored in the memory 27 or may be stored in a server (not illustrated) separate from the drain effluent monitor 20. When the electronic medical record is stored in the server, the drain effluent monitor 20 can be configured to be able to register with the electronic medical record and browse the electronic medical record by communicating with the server via a wireless chip 28.

### (Operation of Effluent Sampling Mechanism)

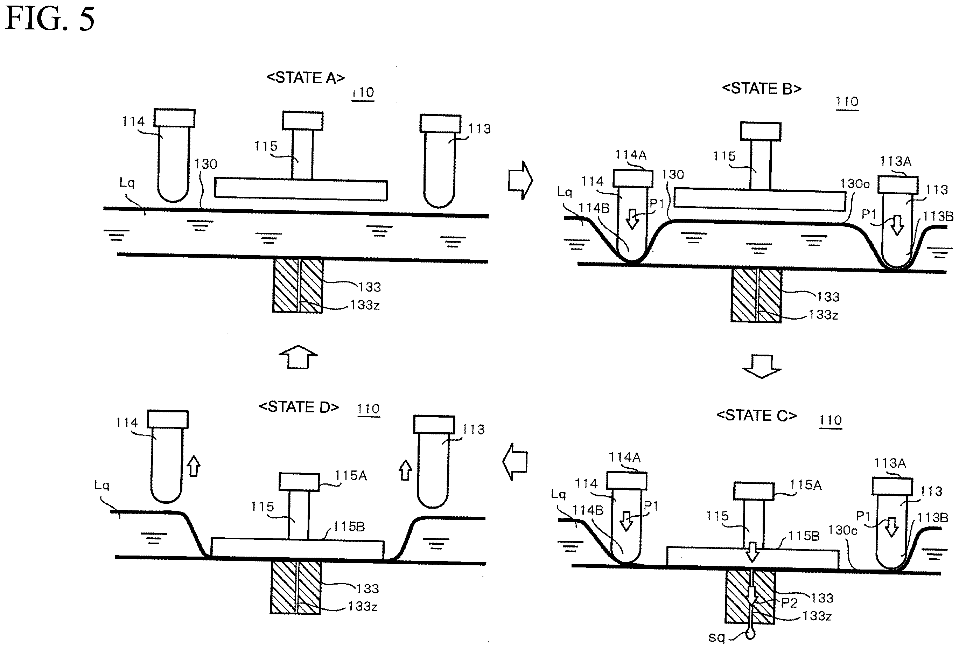

FIG. 5 is a diagram illustrating an operation of the effluent sampling mechanism 110. When the main tube 130 is not pressed, which is a non-pressure state (state A), a liquid (for example, drain effluent or distilled water) flows into the tube of the main tube 130.

First, the restriction members 113 and 114 substantially simultaneously press the main tube 130 to restrict the flow of the liquid (state B). The inside of the tube on both sides of the main tube 130 interposing the branch point, that is, the tube central portion 130c is closed by the partition plates 113B and 114B (state B). As a result, the flow of the liquid inside and outside the tube central portion 130c is restricted. The liquid may slightly flow without being completely closed. Therefore, a certain amount of liquid between the restriction members 113 and 114 stays in the tube central portion 130c. In the state B, the flow path 133z of the sub-tube 133 is closed, and the flow path 133z does not allow the liquid to pass through.

The first pressing member 115 presses the tube central portion 130c of the main tube 130 in a state where the restriction members 113 and 114 substantially simultaneously press the main tube 130 and the liquid stays in the tube central portion 130c of the main tube 130 (state C). When the tube central portion 130c is pressed, the pressure of the liquid staying in the tube central portion 130c of the main tube 130 increases. Due to the increase in the pressure of the liquid, the flow path 133z of the sub-tube 133 connected to the main tube 130 at the branch point expands. The liquid staying in the tube central portion 130c of the main tube 130 flows into the flow path 133z of the sub-tube 133, and flows out from the end surface on a side opposite to the sub-tube 133. When substantially all of the liquid flows out, the tube central portion 130c of the main tube 130 is recessed. As a result, a certain amount of liquid staying between the restriction members 113 and 114 can be fed out through the sub-tube 133. Therefore, in the state C, the flow path 133z of the sub-tube 133 is opened, and the flow path 133z allows the liquid to pass through.

In a state where the first pressing member 115 presses the tube central portion 130c of the main tube 130, the restriction members 113 and 114 stop pressing and depressurize portions on both sides (two portions) interposing the branch point of the main tube 130 (state D). Furthermore, when the first pressing member 115 releases the pressing of the tube central portion 130c, the main tube 130 returns to the state A and is in the non-pressure state. In the state A following the state D, it is possible to prevent the liquid from flowing back from the sub-tube 133 to the main tube 130 due to a decrease in the pressure in the tube central portion 130c.

In addition, when the state changes to the state B after the state A, a new certain amount of liquid flowing through the main tube 130 stays and can be ensured.

By repeating the states A to D, the effluent sampling operation can be continuously performed, and a certain amount of liquid can be extracted. The effluent sampling operation by the effluent sampling mechanism 110 is not limited to the drain effluent Lq, and can be applied to various liquids as a liquid sampling operation by a liquid sampling mechanism.

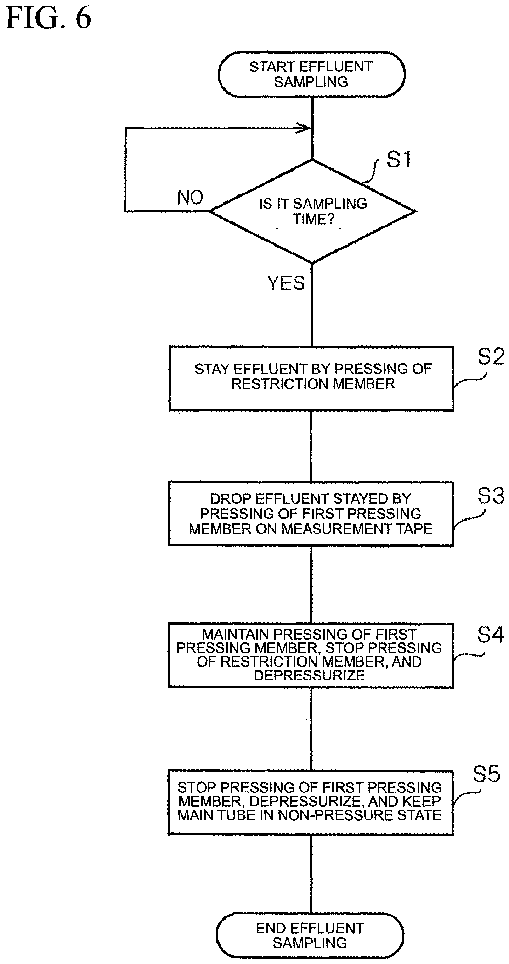

FIG. 6 is a flowchart illustrating an effluent sampling operation procedure by the drain effluent sensor 10. The CPU 181 waits until the sampling time comes (S1). The sampling time is set to an appropriate time, for example, once an hour. When it is the sampling time, the CPU 181 outputs a drive signal to the pressurizing units 113A and 114A, and starts pressing by the restriction members 113 and 114 (S2). When the portions on both sides of the main tube 130 interposing the branch point are pressed by the restriction members 113 and 114, the tube central portion 130c of the main tube 130 is closed by the partition plates 113B and 114B. The liquid stays in the tube central portion 130c of the main tube 130.

The CPU 181 outputs a drive signal to the pressurizing unit 115A while the pressing by the restriction members 113 and 114 maintained, and starts pressing by the first pressing member 115 (S3). When the tube central portion 130c of the main tube 130 is pressed, the pressure of the liquid staying in the tube central portion 130c increases. Due to the increase in the pressure of the liquid, the flow path 133z of the sub-tube 133 expands. The liquid staying in the tube central portion 130c of the main tube 130 passes through the flow path 133z of the sub-tube 133 and flows out from the end surface on the side opposite to the flow path 133z. When substantially all the liquid flows out, the tube central portion 130c of the main tube 130 is recessed.

The CPU 181 outputs a drive signal to the pressurizing unit 115A, stops the drive signal to the pressurizing units 113A and 114A while the pressing operation by the first pressing member 115 maintained, and starts depressurization by the restriction members 113 and 114 (S4). Even when the depressurization is performed by the restriction members 113 and 114, the tube central portion 130c of the main tube 130 is maintained to be recessed.

The CPU 181 stops the drive signal to the pressurizing unit 115A, and starts depressurization by the first pressing member 115 (S5). When the depressurizing operation is performed by the first pressing member 115, the state returns to the non-pressure state, and the liquid flows through the tube central portion 130c of the main tube 130. The direction where the liquid flows is a direction toward the drain bag 40 from the patient side.

According to the operation procedure of the effluent sampling, the drain effluent management system 5 can control the states A to D and easily transition the state of the states A to D. As a result, the drain effluent management system 5 can quantitatively and continuously sample the drain effluent.

### (Measurement of Sampling Liquid)

Next, a method of measuring the amount of the enzyme contained in the drain effluent sampled by the effluent sampling mechanism 110, that is, the sampling liquid sq, will be described.

As described above, the blood cell separation and enzyme reaction mechanism 150 separates blood cells contained in the drain effluent, causes an enzyme to react with a reagent, and optically measures the amount of the enzyme. The blood cell separation and enzyme reaction mechanism 150 receives the sampling liquid sq sampled by the effluent sampling mechanism 110 through the flow path 210 (flow cell, refer to FIG. 7A) included in the measurement tape 200. The flow path 210 feeds at least a portion of the received sampling liquid sq, and supports measurement of the liquid state using the measurement light.

### (Structure of Flow Path)

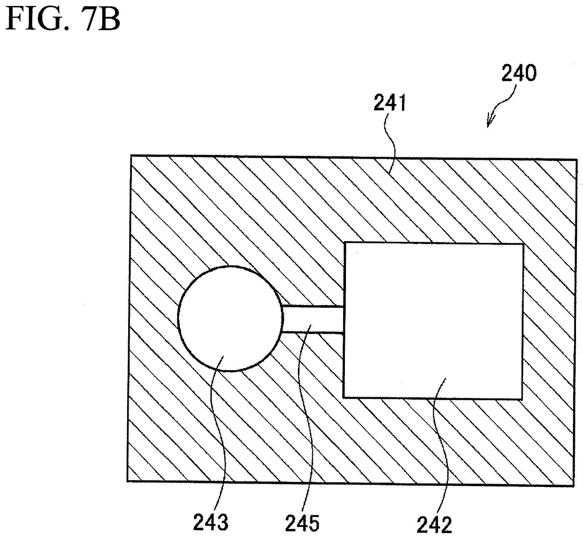

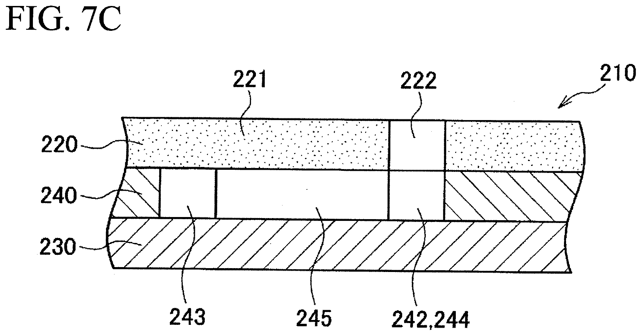

FIG. 7A is an exploded perspective view illustrating an example of the flow path 210 formed in the measurement tape 200. FIG. 7B is a plan view illustrating an example of an adhesive layer 240 in the flow path 210. FIG. 7C is a cross-sectional view illustrating an example of the flow path 210. The flow path 210 introduces the sampling liquid sq and feeds at least a portion of the sampling liquid sq. The fed sampling liquid sq is irradiated with measurement light, and the state of the sampling liquid sq is measured.

The flow path 210 includes a porous sheet 220, an affinity sheet 230, and the adhesive layer 240. The porous sheet 220 may be located on the upper surface of the flow path 210. The affinity sheet 230 may be located on the bottom surface of the flow path 210 and may be a base material. An adhesive 241 included in the adhesive layer 240 may be located on the side surface of the flow path 210.

The porous sheet 220 includes a plurality of fine holes 221 and a through-hole 222. The fine hole 221 serves as a through-hole for maintaining the pressure in the flow path 210 at atmospheric pressure. Therefore, even when the flow path 210 is not provided with an exhaust port, the inside of the flow path 210 does not become a sealed state, and ventilation can be performed by the fine hole 221 of the porous sheet 220. For example, the sampling liquid sq from the effluent sampling mechanism 110 is introduced into the through-hole 222. The porous sheet 220 may have a light-transmitting property with respect to the measurement light (for example, light for measuring amylase or bilirubin, or light having a peak value at a wavelength of 410 nm). The porous sheet 220 is, for example, a porous film.

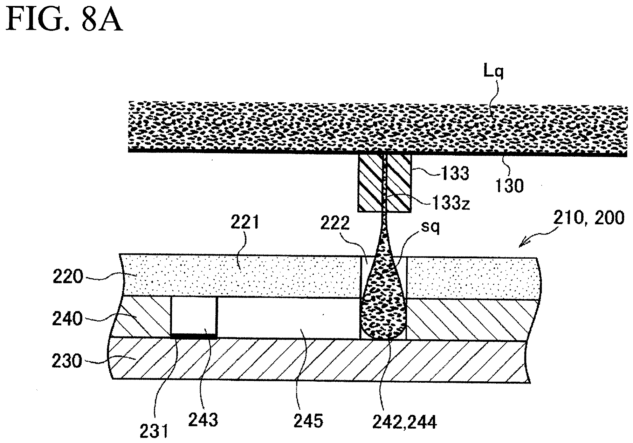

The affinity sheet 230 has an affinity for the sampling liquid sq. The affinity sheet 230 may be an affinity polyethylene terephthalate (PET) sheet, and may be an affinity base material. The affinity sheet 230 may have a light-transmitting property with respect to the measurement light (for example, light for measuring amylase or bilirubin, or light having a peak value at a wavelength of 410 nm). A reagent 231 may be disposed in at least a portion of the surface facing the adhesive layer 240 on the affinity sheet 230 (refer to FIG. 8A and the like).

The adhesive layer 240 is disposed between the porous sheet 220 and the affinity sheet 230. The adhesive layer 240 includes the adhesive 241 for adhering the porous sheet 220 and the affinity sheet 230 to each other. Spaces 242 and 243 and a connection path 245 are defined in a portion where the adhesive 241 is absent in the adhesive layer 240.

The space 242 communicates with the through-hole 222 of the porous sheet 220. A blood cell separation membrane 244 is disposed in the space 242. Therefore, when the sampling liquid sq is dropped, the liquid reaches the blood cell separation membrane 244 disposed in the space 242. The blood cell separation membrane 244 receives the sampling liquid sq introduced into the through-hole 222 and adsorbs a portion of the sampling liquid sq. An adsorption force of the blood cell separation membrane 244 may be different for each component to be managed (for example, blood cells, amylase, and bilirubin). The connection path 245 is defined between the space 242 and the space 243, that is, between the blood cell separation membrane 244 and the space 243. The connection path 245 connects the space 242 and the space 243 to each other and is a space where the liquid actually moves. Therefore, the connection path 245 is inside the flow path 210 where the liquid actually flows.

The sampling liquid sq2 not adsorbed by the blood cell separation membrane 244 flows out to the connection path 245. The sampling liquid sq2 is fed to the space 243 via the connection path 245 by a force caused by affinity for the affinity sheet 230. The force caused by the affinity for the affinity sheet 230 is, for example, a capillary force or surface tension of the affinity sheet 230. The fed sampling liquid sq2 reaches the space 243 and is stored in the space 243. The sampling liquid sq2 stored in the space 243 may react with the reagent 231 disposed on the affinity sheet 230 (refer to FIG. 8C and the like).

The space 243 in which the sampling liquid sq2 is stored is irradiated with the measurement light from the LEDs 182 (for example, LEDs 182A and 182B) of the sensor unit 180. The sampling liquid scatters at least a portion of the irradiated measurement light. The scattered light can be received by a photo sensor 183. The CPU 181 (refer to FIG. 2A) measures the state of the sampling liquid sq2 (for example, amylase) based on the light received by the photo sensor 183.

Next, specific materials of the porous sheet 220, the affinity sheet 230, and the adhesive 241 will be described.

The porous sheet 220 may have the following properties.
Material: polytetrafluoroethylene (PTFE) resin
Thickness: 0. 1 (mm)
Hole diameter: 3 (µm)
Air permeability: 5 (cm³▪ cm⁻²▪ s⁻¹)
Ventilation volume: 302 (cm3/min at 1 kPa)

The hole diameter is a hole diameter of the fine holes 221 of the porous sheet 220. The air permeability is a value measured according to the air permeability measurement method A (Fragile type method) specified in JIS L 1096 (2010). When the porous sheet 220 is used, the flow path 210 can feed the sampling liquid sq2. It is considered that this is because the ventilation is sufficiently performed, so that an increase in the internal pressure in the flow path 210 is suppressed, and the liquid can be fed by a force (for example, surface tension) due to affinity for the sampling liquid sq2. Since the PTFE resin has hydrophobicity, the porous sheet 220 is a hydrophobic sheet.

The following properties are considered as a porous sheet of a comparative example.
Material: PTFE resin
Thickness: 0.1 (mm)
Hole diameter: 0.6 (µm)
Air permeability: 10 (sec/100cm³)
Ventilation volume: 10 (cm³/min at 1 kPa)

The air permeability is a value measured according to the air permeability measurement method B (Gurley-type method) specified in JIS L 1096 (2010). When the porous sheet of the comparative example is used, the flow path 210 cannot feed the sampling liquid sq2. It is considered that this is because the air permeability and ventilation volume of the comparative example are smaller than the air permeability and ventilation volume of the porous sheet 220 whose properties are exemplified, so that the internal pressure in the flow path 210 increases and a force for inhibiting a liquid feeding force due to the surface tension acts.

As the affinity sheet 230, a PET film in which Lumirror type S (for example, Lumirror types S10, S15, S105, S56) manufactured by Toray Industries, Inc. is converted into SiO₂ by sputtering may be used. As the affinity sheet 230, a hydrophilic treatment film # 9901P (hydrophilic PET film using a surfactant) manufactured by 3M Japan Ltd. may be used. Each of the affinity sheets 230 has hydrophilicity (affinity for the sampling liquid sq). The flow path 210 can be fed even when the sampling liquid sq contains a high-protein and high-viscosity liquid such as plasma due to the affinity of the affinity sheet 230.

The adhesive 241 may have properties of any one of the following adhesives (1), (2), and (3).

### <Adhesive (1)>

Material: Acrylic adhesive
Thickness: 0.2 (mm)

When the adhesive (1) is used, since the acrylic adhesive has hydrophobicity (non-affinity for sampling liquid sq), the liquid leakage in which the sampling liquid sq2 leaks from the flow path 210 does not occur. In addition, since the thickness of the adhesive (1) is small, the liquid feeding speed of the sampling liquid sq2 is high. In addition, the adhesive (1) has a relatively high elasticity and thus is not easily deformed, and the adhesive does not easily adhere to a blade used for punching the adhesive, so that the handling is good. This handling corresponds to the adhesion performance between the porous sheet 220 on the upper surface and the affinity sheet 230 on the bottom surface. Therefore, when the handling is good, it indicates that the adhesive performance is high.

### <Adhesive (2)>

Material: Acrylic adhesive
Thickness: 0.2mm

When the adhesive (2) is used, since the acrylic adhesive has hydrophobicity (non-affinity for sampling liquid sq), the liquid leakage in which the sampling liquid sq2 leaks from the flow path 210 does not occur. In addition, since the thickness of the adhesive (2) is small, the liquid feeding speed of the sampling liquid sq2 is high. In addition, the adhesive (2) has a relatively small elasticity and thus is easily deformed, and the adhesive easily adheres to a blade used for punching the adhesive, so that the handling is slightly inferior to adhesive (1).

### <Adhesive (3)>

Material: Acrylic adhesive
Thickness: 0.4mm

When the adhesive (3) is used, since the acrylic adhesive has hydrophobicity (non-affinity for sampling liquid sq), the liquid leakage in which the sampling liquid sq2 leaks from the flow path 210 does not occur. In addition, since the thickness of the adhesive (3) is larger than that of the adhesives (1) and (2), the liquid feeding speed of the sampling liquid sq2 is lower than that of the adhesives (1) and (2). In addition, since the thickness of the adhesive (3) is larger than that of the adhesives (1) and (2), the adhesive may be crushed and the inside of the flow path 210 may be closed, so that the handling is inferior to adhesive (1) and (2).

As the adhesive of the comparative example, an adhesive which is an acrylic adhesive and has a thickness of 0.1 mm can be considered. When the adhesive of the comparative example is used, the liquid leakage of the sampling liquid sq2 leaks from the flow path 210 occurs. This is considered to be due to the insufficient thickness of the adhesive, and due to the formation of a space between the adhesive 241 and the blood cell separation membrane 244, and leakage of blood cells (that is, sampling liquid sq1) from this space.

When the adhesive has strechability, the adhesive has high adhesion to the porous sheet 220 and the affinity sheet 230 and is hard to be peeled off, whereas easily leaks liquid and the like, and the liquid feeding performance is reduced. In addition, when the adhesive does not have strechability, the adhesive has low adhesion to the porous sheet 220 and the affinity sheet 230 and is easily peeled off, whereas hardly leaks liquid and the like, and the liquid feeding performance is improved. As described above, there is a trade-off relationship between the adhesion of the adhesive and the liquid feeding performance.

Next, a liquid feeding experiment of the sampling liquid sq2 will be described. In the liquid feeding experiment, various members were used for a first sheet provided on the upper surface of the flow path 210 and a second sheet provided on the lower surface of the flow path 210. In this liquid feeding experiment, in the flow path 210, the diameter of the hole of the space 243 was 6 mm, the length of the connection path 245 was 2 mm, the width of the connection path 245 was 2 mm, the blood cell separation membrane 244 was MF1, the size thereof was 15 mm x 10 mm, and the thickness of the adhesive 241 (adhesive layer 240) was 0.2 mm. MF1 is a glass membrane, and has the properties that the thickness thereof (thickness measured under a pressure of 53 kPa applied to the glass membrane) is 367 µm, the suction amount thereof is 29.7 s/4 cm, and the water supply amount thereof is 39.4 mg/cm².

The experimental results in the liquid feeding experiment were as follows. The description that "liquid could not be fed" means that the sampling liquid sq2 did not reach the space 243 in an amount of the threshold th or larger. The description that "liquid could be fed" means that the sampling liquid sq2 did reached the space 243 in an amount of the threshold th or larger. Here, the liquid feeding experiments of six patterns were performed using a combination of the first sheet and the second sheet.

### <Pattern 1>

First sheet: non-affinity PET sheet (non-porous sheet)
Second sheet: non-affinity PET sheet
Determination result: liquid could not be fed

In Pattern 1, it is considered that the internal pressure of the flow path 210 increased without being maintained at the atmospheric pressure because the flow path 210 was not provided with a porous sheet, and the liquid could not be fed because a force hindering the liquid feeding was applied.

### <Pattern 2>

First sheet: affinity PET sheet (non-porous sheet)
Second sheet: non-affinity PET sheet
Determination result: liquid could not be fed

In the pattern 2, it is considered that the internal pressure of the flow path 210 increased without being maintained at the atmospheric pressure because the flow path 210 was not provided with a porous sheet, and the liquid could not be fed because a force hindering the liquid feeding was applied.

### <Pattern 3>

First sheet: non-affinity PET sheet (porous sheet)
Second sheet: affinity PET sheet
Determination result: liquid could be fed

In Pattern 3, it is considered that the internal pressure of the flow path 210 was maintained at the atmospheric pressure without increasing, and the force hindering the liquid feeding was suppressed because the flow path 210 was provided with the porous sheet. It is considered that the liquid feeding force was obtained by the force caused by the affinity between the affinity PET sheet and the liquid. Therefore, it is considered that the liquid could be fed as a result.

### <Pattern 4>

First sheet: affinity PET sheet (porous sheet)
Second sheet: affinity PET sheet
Determination result: liquid could be fed, and liquid leaked

In Pattern 4, it is considered that the internal pressure of the flow path 210 was maintained at the atmospheric pressure without increasing, and the force hindering the liquid feeding was suppressed because the flow path 210 was provided with the porous sheet. In addition, it is considered that the liquid feeding force was obtained by the force caused by the affinity between the affinity PET sheet and the liquid. Therefore, it is considered that the liquid could be fed as a result. In addition, it is considered that the liquid permeated the affinity PET sheet and leaked because the flow path 210 was not provided with a sheet having non-affinity.

### <Pattern 5>

First sheet: affinity PET sheet (porous sheet)
Second sheet: non-affinity PET sheet
Determination result: liquid could not be fed

In Pattern 5, it is considered that the liquid could not be fed because the second sheet had non-affinity, and a contact angle of the liquid to the second sheet did not exceed 90 degrees. In addition, it is considered that the liquid is fed by the force caused by the sum of the affinity of the first sheet and the affinity of the second sheet. In the pattern 5, it is considered that the liquid could not be fed because non-affinity was superior to affinity.

### <Pattern 6>

First sheet: non-affinity PET sheet (porous sheet)
Second sheet: non-affinity PET sheet
Determination result: liquid could not be fed

In the pattern 6, it is considered that a force caused by the affinity between the affinity PET sheet and the liquid could not be obtained, the liquid feeding force could not be obtained, and the liquid could not be fed, because the flow path 210 was not provided with the affinity PET sheet.

When the affinity PET sheet is used as the first sheet, as an example, a sheet in which a monolith membrane is subjected to a hydrophilic treatment may be used.

Next, the movement of the sampling liquid sq in the flow path 210 will be described.

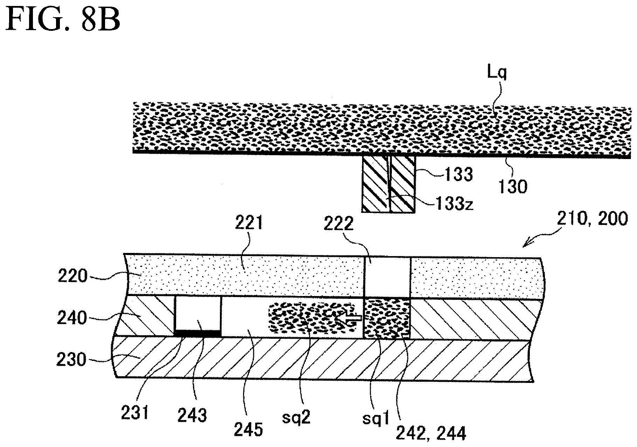

FIGS. 8A, 8B, and 8C are views illustrating a state where the blood cell separation and enzyme reaction is performed using the measurement tape 200. FIG. 8A is a cross-sectional view illustrating an example of introduction of the sampling liquid sq into the flow path 210 of the measurement tape 200. FIG. 8B is a cross-sectional view illustrating an example of movement of the sampling liquid sq separated by the blood cell separation membrane 244 in the flow path 210. FIG. 8C is a cross-sectional view illustrating an example in which the sampling liquid sq2 is reached the space 243 in the flow path 210 at a liquid feeding destination.

The measurement tape 200 including the flow path 210 has a three-layer structure. The three-layer structure includes the affinity sheet 230 disposed on the lowermost layer, the adhesive layer 240 disposed thereon further, and the porous sheet 220 disposed on the uppermost layer. The flow path 210 is included in the measurement tape 200. In the measurement tape 200, the through-holes 222 of the porous sheet 220, the spaces 242 and 243 of the adhesive layer 240, the blood cell separation membrane 244, the connection path 245, and the like are repeatedly formed along the measurement tape 200. That is, the flow path 210 illustrated in FIG. 8A is repeatedly formed along the measurement tape 200.

The affinity sheet 230 may stack the reagent 231 and form an enzyme reaction sheet with the affinity sheet 230 and the reagent 231. In this case, the reagent 231 may be dropped on the affinity sheet 230, dried, and transferred. When the sampling liquid sq2 reaches the position where the reagent 231 is transferred, the reagent 231 dissolves, and the sampling liquid sq (for example, amylase) and the reagent 231 react.

The blood cell separation membrane 244 is capable of separating blood cells from non-blood cells (for example, amylase) based on the size of the component to be managed (for example, blood cells, amylase, and bilirubin) and adsorption of the component to be managed contained in the drain effluent.

As illustrated in FIG. 8A, the sampling liquid sq flowing out from the flow path 133z of the sub-tube 133 is introduced through the through-hole 222 of the porous sheet 220, and reaches the blood cell separation membrane 244 disposed in the space 242 of the adhesive layer 240 communicating with the through-hole 222.

The blood cell separation membrane 244 is made of, for example, a glass membrane. The glass membrane is a sheet shape made by bundling glass fibers, and is formed like a nonwoven fabric. The glass membrane may be formed in a sheet shape by overlapping and folding multiple glass fibers.

The blood cell separation membrane 244 adsorbs the component to be managed. An adsorption force of the blood cell separation membrane 244 may be different for each component to be managed (for example, blood cells, amylase, and bilirubin). In addition, the adsorption force of the blood cell separation membrane 244 may be changed depending on the surface area of the blood cell separation membrane 244. The size of the surface area of the blood cell separation membrane 244 may be determined according to the density of the blood cell separation membrane 244. The glass membrane may be charged and adsorbed. Specifically, the glass fibers may be positively charged, blood cells (for example, phospholipids of blood cells) may be negatively charged in the sampling liquid, and both may be electrically attracted. On the other hand, the adsorption force between the glass fiber and the enzyme in the sampling liquid may be weaker than the adsorption force between the glass fiber and the blood cell. In this case, the enzyme may move on the glass fiber, flow out to the outside of the blood cell separation membrane 244, and proceed to the connection path 245.

When the sampling liquid sq flowing out from the flow path 133z of the sub-tube 133 is dropped on the blood cell separation membrane 244, blood cells are adsorbed to the blood cell separation membrane 244. Therefore, the sampling liquid sq1 (blood cell component) containing a large amount of blood cells stays in the vicinity of the dropping position of the blood cell separation membrane 244.

On the other hand, when the sampling liquid sq2 containing a large amount of an enzyme (for example, amylase) is dropped on the blood cell separation membrane 244, the sampling liquid sq2 is not immediately adsorbed at the dropping position of the blood cell separation membrane 244, but spreads from the dropping position and moves leftward, for example, as illustrated in FIG. 8B. A portion of the sampling liquid sq2 permeates into the connection path 245 without staying in the blood cell separation membrane 244. Therefore, the sampling liquid sq1 containing a large amount of blood cells stays in the vicinity of the dropping position of the blood cell separation membrane 244, and the sampling liquid sq2 containing a large amount of amylase moves to a position away from the dropping position of the blood cell separation membrane 244 (for example, end portion of the blood cell separation membrane 244 or connection path 245).

As described above, in the blood cell separation membrane 244, the sampling liquid sq is separated due to the difference in the adsorption force for each component in the sampling liquid sq. As a result, blood cells as the sampling liquid sq1 stay on the blood cell separation membrane 244, and non-blood cells (amylase or bilirubin) as the sampling liquid sq2 permeate from the blood cell separation membrane 244.

As illustrated in FIG. 8B, the sampling liquid sq2 permeated from the blood cell separation membrane 244 enters the connection path 245. The connection path 245 (in the flow path 210) is maintained at the same pressure as the atmospheric pressure by the fine holes 221 of the porous sheet 220 located on the upper surface of the flow path 210. The connection path 245 feeds the sampling liquid sq2 toward the space 243 by a force (for example, surface tension of the affinity sheet 230) caused by the affinity of the affinity sheet 230 located on the lower surface of the connection path 245.

As illustrated in FIG. 8C, the sampling liquid sq2 fed via the connection path 245 reaches the space 243. The space 243 may be disposed to face the reagent 231 attached (for example, transferred) to the affinity sheet 230. The sampling liquid sq2 reached the space 243 reacts with the reagent 231. For example, when the enzyme is an amylase, the reagent 231 reacts with the amylase and changes the amylase to yellow. In addition, the amylase reacted with the reagent 231 absorbs a portion of the measurement light. The reagent 231 may be, for example, an amylase activity measurement reagent used in a commercially available dry clinical chemistry analyzer.

The amylase reacted with the reagent 231 easily absorbs light having a wavelength of 410 nm. On the other hand, the absorption wavelength of blood cells is 420 nm. Thus, when the measurement light having a peak value at 410 nm is projected onto the amylase reacted with the reagent 231, and the absorbance of the amylase is measured from the scattered light based on the measurement light, the measurement overlaps the absorbance wavelength of the blood cells of 420 nm, and accurate measurement is difficult. Thus, the blood cell separation and enzyme reaction mechanism 150 separates into blood cells and non-blood cells (for example, amylase and bilirubin) and measures the absorbance and the like.

Therefore, the sensor unit 180 measures the absorbance of the amylase contained in the sampling liquid sq2 permeated the measurement tape 200 fed out by the tape winding and feeding mechanism 170. On measuring the absorbance of the amylase, the LED 182 of the sensor unit 180 projects the measurement light having a peak value at 410 nm, which is the absorption wavelength, onto the measurement tape 200 (specifically, space 243 where sampling liquid sq2 is located) into which the sampling liquid sq2 is permeated. The photo sensor 183 of the sensor unit 180 receives scattered light projected from the LED 182 without being absorbed by amylase in the sampling liquid sq2. The CPU 181 of the sensor unit 180 measures the absorbance of amylase contained in the sampling liquid sq2 based on the amount of scattered light received by the photo sensor 183. The CPU 181 estimates the amylase activity based on the absorbance of the amylase. The amylase activity represents the capacity of amylase to react with reagent 231 and is expressed in units of U/L. The CPU 181 calculates the amylase activity based on, for example, the amount of change in absorbance ΔA of the amylase.

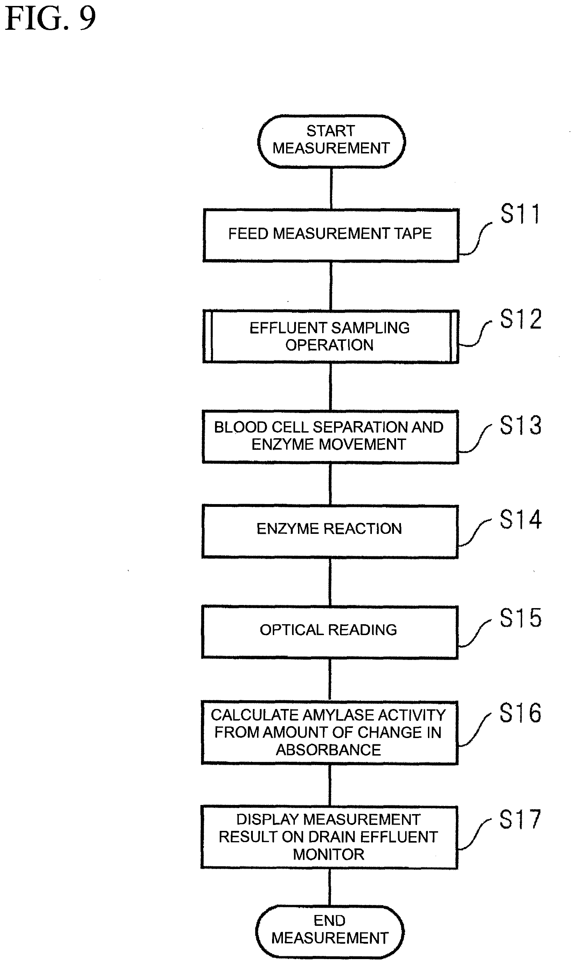

FIG. 9 is a flowchart illustrating a procedure for measuring amylase activity by the drain effluent management system 5. This measurement is set at an appropriate time, for example, once an hour, similarly to the effluent sampling operation.

The CPU 181 of the sensor unit 180 outputs a command signal via the motor drive unit 187, and drives the motor 175 so as to feed the measurement tape 200 in the winding direction (S11). When the motor 175 rotates, the winding reel 172 rotates and winds the measurement tape 200 so that the through-hole 222 of the flow path 210 serving as a dropping position is located directly below the sub-tube 133 (facing position) in order to perform the effluent sampling operation, and the feed reel 151 feeds out the measurement tape 200.

The CPU 181 performs the effluent sampling operation, and drops the sampling liquid sq into the through-hole 222 of the flow path 210 of the measurement tape 200 (S12). This effluent sampling operation may be performed according to the procedure illustrated in the flowchart of FIG. 6.

The CPU 181 waits for a predetermined time to perform the blood cell separation and the movement of the sampling liquid sq. During the waiting for the predetermined time, the sampling liquid sq dropped onto the blood cell separation membrane 244 via the through-hole 222 of the measurement tape 200 is separated into a blood cell component and a non-blood cell component by the blood cell separation membrane 244 (S13). The sampling liquid sq1 containing a large amount of blood cells of the sampling liquid sq stays in the vicinity of the dropping position, and the sampling liquid sq2 containing amylase moves to a portion away from the dropping position and enters the connection path 245 (S13). The sampling liquid sq2 having entered the connection path 245 moves by a force (for example, surface tension) due to affinity for the affinity sheet 230, and reaches the space 243 (S13).

The CPU 181 waits for the reaction time between the amylase and the reagent 231. During this reaction time, the amylase contained in the sampling liquid sq2 having reached the space 243 reacts with the reagent 231 located at a position facing the space 243, and the reacted amylase turns yellow (S14).

The CPU 181 performs optical reading on the reaction portion between the amylase and the reagent 231 (S15). In this optical reading, the CPU 181 turns on the LED 182 (for example, at least one of the LEDs 182A and 182B) and projects the measurement light toward the reaction portion. At the reaction portion, a portion of the projected measurement light is absorbed by the amylase, and the remaining portion is scattered. The CPU 181 receives the light scattered by the photo sensor 183 and calculates the amount of change in absorbance (ΔA) based on the amount of light received.

The CPU 181 calculates the amylase activity based on the amount of change in the absorbance (ΔA) (S16). The CPU 181 communicates with the drain effluent monitor 20 by the wireless chip 184, and transmits measurement data on amylase (for example, the amount of change in absorbance (ΔA) of amylase, the value of amylase activity, and the value of amylase concentration) with information on the measurement time.

When the CPU 26 of the drain effluent monitor 20 receives the measurement data and the information on the measurement time on the amylase from the drain effluent sensor 10 via the wireless chip 28, various data (measurement data, measurement time, other data) is displayed via the display 21 (S17).

According to such a measurement procedure of such a component to be managed (for example, amylase), the drain effluent management system 5 can automatically measure the state of the sampling liquid sq from which the drain effluent is sampled, and can derive the measurement data by the CPU 181 controlling each part of the blood cell separation and enzyme reaction mechanism 150. In addition, the drain effluent management system 5 can display information on the drain effluent monitor 20 and can visualize information on the component to be managed such as measurement data. Therefore, the user can easily grasp the patient's recovery tendency.

In addition, the drain effluent sensor 10 measures amylase using the blood cell separation membrane 244, so that a large-sized apparatus such as a centrifugal separator is not required, the portability is good, and the cost can be reduced.

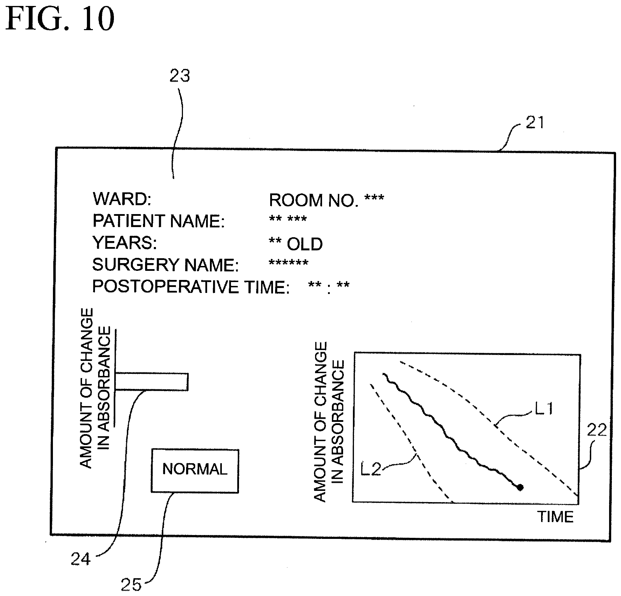

FIG. 10 is a diagram illustrating a display on the drain effluent monitor 20. As an example, the display 21 disposed on the front surface of the drain effluent monitor 20 may display a graph 22 illustrating the measurement result of the amount of change in absorbance (ΔA) representing the amylase activity. In addition, the display 21 may also display various explanatory texts 23 such as the patient's name, a meter 24 indicating the amount of change in absorbance (ΔA), and the status marker 25 for notifying the condition of the patient (normal or abnormal).

In the graph 22, a broken line L1 indicates an upper limit of a normal range, and a broken line L2 indicates a lower limit of the normal range. When the condition of the patient after the operation is normal, the amount of change in absorbance due to amylase in the drain effluent flowing through the drain tube 30 connected to the patient's body gradually decreases as time elapses after the operation. In this example, the temporal transition of each measurement result is maintained within the normal range. At this time, the character "normal" is displayed as the status marker 25. On the other hand, when the temporal transition of each measurement result is out of the normal range, the character "abnormal" may be displayed as the status marker 25.

### (Measurement of Bilirubin)

In the above description, amylase, which is a digestive enzyme, is mainly described as a component to be managed in the drain effluent Lq. The bilirubin contained in bile, urine, and the like can be one of the components to be managed as an example of secretions of the organ. Since the bilirubin itself has a yellow pigment, the concentration of bilirubin can be optically detected without contact. Thus, when the concentration of bilirubin is measured, it is unnecessary to allow it to react with the reagent 231, and the reagent 231 is unnecessary. Except that the reagent 231 is not included, bilirubin may be measured using the measurement tape 200 having the flow path 210 described in FIG. 7A and the like.

The bilirubin can be subjected to membrane separation similarly to amylase. That is, the bilirubin contained in the sampling liquid sq as an example of non-blood cells has lower affinity for the blood cell separation membrane 244 than that of blood cells, and is unlikely to be adsorbed by the blood cell separation membrane 244 compared to the blood cells. Therefore, the bilirubin moves along the blood cell separation membrane 244 by, for example, capillary action, and enters the connection path 245. The bilirubin moves toward the space 243 by a force (for example, surface tension) due to affinity for the affinity sheet 230 in the connection path 245, reaches the space 243, and is stored.

In the membrane measurement, when the sampling liquid sq is dropped on the blood cell separation membrane 244, the blood cells are preferentially adsorbed to the blood cell separation membrane 244 due to the difference in adsorption force between the blood cells and bilirubin. That is, the adsorption force of blood cells to the blood cell separation membrane 244 is greater than the adsorption force of bilirubin to the blood cell separation membrane 244. In the blood cell separation membrane 244, the blood cells are adsorbed in the vicinity of the dropping position, and at least a portion of the bilirubin is not adsorbed and permeates into the connection path 245. The permeated bilirubin reaches the space 243 and is stored. The sensor unit 180 can measure the absorbance of bilirubin by measuring the space 243 where the stored bilirubin exists using light having a wavelength of 410 nm as a peak value as the measurement light.

Therefore, the drain effluent sensor 10 measures bilirubin using the blood cell separation membrane 244, so that a large-sized apparatus such as a spectrophotometer is not required, the portability is good, and the cost can be reduced.

The measurement data such as the absorbance of bilirubin may be sent to the drain effluent monitor 20 and displayed on the display 21.

### (Modified Example of Measurement Tape)

The measurement tape 200 may be configured such that a flow path 210 containing a reagent 231 used for measurement of amylase (flow path 210 for measurement of amylase) and a flow path 210 not containing a reagent 231 used for measurement of bilirubin (flow path 210 for measurement of bilirubin) are alternately included. That is, the measurement tape 200 may be manufactured so that the flow path 210 for measurement of amylase and the flow path 210 for measurement of bilirubin are repeated in the length direction of the measurement tape 200. When the state of amylase is measured, the drain effluent sensor 10 uses the flow path 210 for measurement of amylase in the measurement tape 200. When the state of bilirubin is measured, the drain effluent sensor 10 uses a flow path 210 for measurement of bilirubin in the measurement tape 200.

As described above, the drain effluent sensor 10 uses the measurement tape 200 having the flow path 210 for measurement of amylase and the flow path 210 for measurement of bilirubin, and thus the types of the substances that can be simultaneously measured by the drain effluent sensor 10 can be increased. In addition, since the drain effluent sensor 10 can be shared in the measurement of a plurality of components to be managed different from each other, the cost is reduced and the space can be saved as compared with the case where two drain effluent sensors are provided. In addition, in measuring a plurality of components to be managed different from each other, only one measurement tape 200 is required, so that the cost is reduced and the space can be saved as compared with the case where two measurement tapes 200 are used.

Hereinbefore, although the embodiment is described with reference to the drawings, it goes without saying that the present invention is not limited to such examples. It is apparent to those skilled in the art that various changes or modifications may be made within the scope of the aspects, and it is understood that these also fall within the technical scope of the present invention. In addition, each component in the above embodiment may be randomly combined without departing from the spirit of the invention.

In the above embodiment, the drain effluent discharged from the living body via the drain tube 30 is exemplified as a target of sampling or measurement. A liquid other than the drain effluent may be a target of sampling or measurement. For example, a biological fluid discharged from or introduced into a living body through a body fluid guide tube may be a target of sampling or measurement.

The body fluid guide tube may include, for example, a drain (drain tube), a catheter (catheter tube), and a medication tube (tube used for medication). The biological fluid flows through the body fluid guide tube.

The drain may include drains for cranial nerves, otolaryngology, respiratory organ, circulatory organ, mammary gland and endocrine, upper digestive tract, biliary hepatopancreas, urology, gynecology, and orthopedics. The drain for cranial nerves may include a ventricular drain, a cisternal drain, an epidural drain, a subcutaneous drain, a hematoma cavity drain, a lumbar drain, and a drain used after endoscopic surgery. The drain for otolaryngology may include a drain used after head and neck surgery. The drain for respiratory organ may include a thoracic drain, and a mediastinal drain. The drain for circulatory organ may include a pericardial drain, and a drain used after head and neck surgery. The drain for mammary gland and endocrine may include a drain used after breast cancer surgery, a mastitis drain, and a drain used after thyroid surgery. The drain for upper digestive tract may include a thoracic and mediastinal drain, a cervical drain, an abdominal drain, an abdominal peritonitis drain, an intraperitoneal abscess drain, and a drain used after gastric surgery. The drain for biliary hepatopancreas may include a percutaneous transhepatic gallbladder drain, a percutaneous transhepatic biliary drain, a liver abscess drain, an endoscopic biliary drain, a drain for acute pancreatitis, a lower gastrointestinal tract retroperitoneal abscess drain, a drain used after rectal cancer surgery, and a perianal abscess drain. The drain for urology may include a drain used after general surgery, a drain used after endoscopic surgery, and a drain used for percutaneous and transurethral approaches. The drain for gynecology may include a drain used after open surgery, and a drain used after endoscopic surgery. The drain for orthopedics may include a joint cavity drain. In addition, as other drains, an incision effluent drain may be included.

The catheter may include an angiography catheter, a balloon catheter, a heart catheter, a cerebral vascular catheter, a catheter used for cancer catheter treatment, a vascular indwelling catheter, and a urethral catheter.

The medication tube may include a tube used for a medication device (medication system). The medication device may include a medication pump (target controlled infusion (TCI) pump) that directly controls the drug concentration in the blood. The TCI pump controls the operation of the pump to adjust the administration speed of the drug, and controls the blood concentration of the drug to be a target blood concentration.

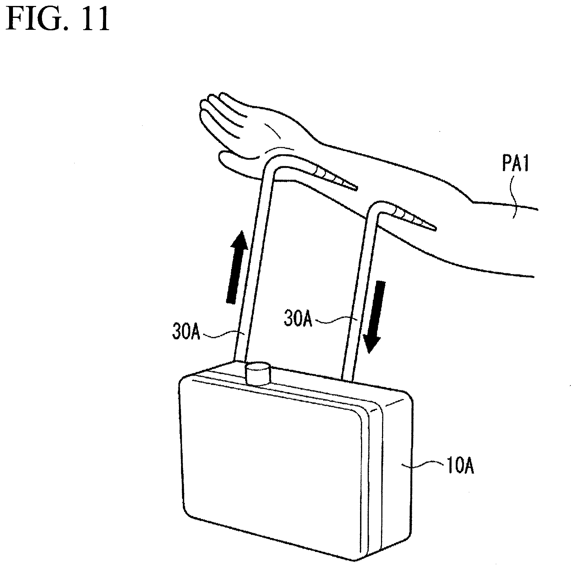

FIG. 11 is a diagram illustrating introduction of a biological fluid containing a drug or an infusion (hereinafter, also referred to as a drug or the like) into the patient PA1 by a TCI pump 10A. A biological fluid containing a drug or the like is introduced from the TCI pump 10A to a patient PA1 via a medication tube 30A. In addition, the biological fluid is fed from the patient PA1 to the TCI pump 10A via the medication tube 30A. That is, the TCI pump 10A adjusts the dose and the administration speed of the drug to the patient PA1, circulates the biological fluid with the patient PA1, and controls the drug concentration in body of the patient PA1. With such a TCI pump 10A, for example, a medication system that directly controls the drug concentration in the blood of the patient PA1 can be realized.

The TCI pump 10A may have the same configuration as the drain effluent sensor 10 except for the configuration related to the control of the administration of the drug to be administered to the patient PA1, and for example, may be the same as the configuration illustrated in FIG. 2. The TCI pump 10A may sample the biological fluid flowing from the TCI pump 10A to the patient PA1 and measure the component to be managed. In addition, the TCI pump 10A may sample the biological fluid flowing from the patient PA1 to the TCI pump 10A, and may measure the component to be managed.

In the above embodiment, an example in which the adhesive layer 240 is provided between the porous sheet 220 and the affinity sheet 230 in the flow path 210 is described. Contrarily, the adhesive layer 240 may not be provided. For example, the porous sheet 220 and the affinity sheet 230 may be integrally formed. In this case, the flow path 210 may be defined by, for example, a micro electro mechanical systems (MEMS) technology.

For example, in a material (for example, sheet) corresponding to the affinity sheet 230, a recessed portion recessed in a shape corresponding to the flow path 210 is formed in a thickness direction (direction where the porous sheet 220 and the affinity sheet 230 are arranged). Thus, the inside of the flow path 210 (for example, spaces 242 and 243 and connection path 245) may be defined. The inside of the defined flow path 210 is subjected to amphiphilic treatment, and a material corresponding to the porous sheet 220 (for example, sheet of a gas permeable membrane) is attached to the material corresponding to the affinity sheet 230. Accordingly, the entire flow path 210 may be formed. The amphiphilic treatment is a treatment for imparting an affinity to the liquid flowing through the flow path 210. The gas permeable membrane may have a non-affinity for the liquid to be fed.

Similarly, in a material (for example, sheet) corresponding to the porous sheet 220, a recessed portion recessed in a shape corresponding to the flow path 210 is formed in a thickness direction (direction where the porous sheet 220 and the affinity sheet 230 are arranged). Thus, the inside of the flow path 210 (for example, spaces 242 and 243 and connection path 245) may be defined. Then, a material corresponding to the affinity sheet 230 (for example, a sheet) is attached to the material corresponding to the porous sheet 220, so that the entire flow path 210 may be formed. A surface of the affinity sheet 230 facing the inside of the flow path 210 may be subjected to the amphiphilic treatment.

In the above embodiment, an example in which the porous sheet 220 is disposed on the upper surface of the flow path 210 and the affinity sheet 230 is disposed on the lower surface of the flow path 210 is described, and the reverse may be applied. That is, the porous sheet 220 may be disposed on the lower surface of the flow path 210, and the affinity sheet 230 may be disposed on the upper surface of the flow path 210. In this case, it is considered that the same effect as that of the present embodiment can be obtained.

In the above embodiment, an example in which the blood cell separation membrane 244 separates the sampling liquid sq into a blood cell component and a non-blood cell component is described. However, the embodiment is not limited thereto. For example, the separation membrane may separate a liquid other than the sampling liquid sq (for example, chemical material) into a plurality of components other than a blood cell component and a non-blood cell component.

In the above embodiment, an example relating to sampling of a liquid is disclosed. Contrarily, the embodiment is not limited thereto. For example, the embodiment can be applied to uses such as liquid dispensing and dropping, in which liquid is discharged at a constant volume.

In the above embodiment, an example in which the sampling is performed by the effluent sampling mechanism 110 is described. Contrarily, the effluent sampling mechanism 110 may be omitted. In this case, the liquid may be dropped directly into the flow path 210. In addition, the separation membrane may not be provided, and all of the dropped liquids may be measured. For example, a liquid that is not separated (one type of component) can also be measured using the measurement light.

In the above embodiment, an example in which a glass membrane is mainly used as the blood cell separation membrane 244 is described. Contrarily, other members may be used. For example, a positively charged membrane that can be charged and adsorbed to blood cells may be used, or a member having an adsorption mechanism other than the charged adsorption may be used.

In the above embodiment, an example is described in which the data of each component to be managed in the sampling liquid sq is acquired in a time series. In addition to these time-series data, time-series data of the total discharge amount of the drain effluent Lq and the sampling liquid sq may be acquired. The time-series data of the total discharge amount of the drain effluent Lq may be acquired based on, for example, an output value of a sensor (for example, strain sensor) that measures the weight of the drain bag. The time-series data of the total discharge amount of the sampling liquid sq may be acquired, for example, by measuring the extraction amount of the sampling liquid sq every time by the effluent sampling mechanism 110 and integrating each time. In addition, the time-series data may include data on the discharge amount of the drain effluent Lq and the sampling liquid sq per unit time.

In the above embodiment, the CPU 181 of the drain effluent sensor 10 may calculate the amylase concentration based on the amount of change in absorbance of the amylase. Amylase concentration data is an example of measurement data. For example, correspondence information on the amount of change in absorbance of amylase and the concentration of amylase may be stored in a memory or the like, and the concentration of amylase may be derived based on this correspondence information. The CPU 181 of the drain effluent sensor 10 may calculate the concentration of bilirubin based on the absorbance of bilirubin. The data of the concentration of bilirubin is an example of measurement data. For example, correspondence information on the absorbance of bilirubin and the concentration of bilirubin may be stored in a memory or the like, and the concentration of bilirubin may be derived based on this correspondence information.

In the above embodiment, the drain effluent sensor 10 and the drain effluent monitor 20 are configured as separate devices. Contrarily, the drain effluent sensor 10 and the drain effluent monitor 20 may be configured as devices having the same case.

In the above embodiment, an example in which the drain tube 30 is connected to the main tube 130 is described. Contrarily the main tube 130 may be a portion of the drain tube 30.

In the above embodiment, the blood cell separation and enzyme reaction mechanism 150 of the drain effluent sensor 10 may measure a blood concentration contained in the sampling liquid sq as the component to be managed. For example, the blood concentration in the sampling liquid sq can be measured as follows. For example, it is possible to derive the blood concentration in the sampling liquid sq by irradiating the space 242 where the blood cell separation membrane 244 to which blood cells are adsorbed is disposed with the measurement light and receiving the scattered light. As the measurement light for measuring the blood concentration, for example, light having a peak value of a wavelength of 660 nm or 850 nm may be used. That is, the LED 182B that emits infrared light may be used. As described above, the biological fluid (for example, component to be managed) may include blood cells of a living body (for example, venous blood of patient). By measuring the patient's venous blood, the drain effluent sensor 10 can evaluate the state of the patient based on the state of the blood.

As described above, the drain effluent management system 5 performs collection, separation, dispensing, analysis, and the like of the drain effluent Lq discharged from the living body of the patient, and can provide the user with an index for examining the subsequent treatment for the patient. In addition, collection, separation, dispensing, analysis, and the like of the drain effluent Lq can be performed by the drain effluent sensor 10 as one device.

In addition, it is assumed that the patient wears the drain tube 30 on the patient's body, for example, for approximately one week. The frequency of sampling the drain effluent Lq may be, for example, approximately once an hour, or approximately 170 times a week.

As described above, the flow path 210 is a flow path 210 for feeding a liquid, and at least a portion of the flow path 210 includes a first region having a plurality of fine holes 221 (example of first through-hole) for maintaining the pressure in the flow path 210 at atmospheric pressure and a second region having an affinity for the liquid. The liquid is fed by a force caused by affinity for the second region. The first region is a region including the porous sheet 220 in the flow path 210, for example. The second region is a region including the affinity sheet 230 in the flow path 210, for example.

As a result, even when there is no porous member at a portion through which the liquid passes and there is no capillary force generated in the porous member as a drive force for liquid feeding, the flow path 210 can feed the liquid by a force (for example, surface tension) caused by the affinity of the second region. In addition, the flow path 210 allows air to pass through the fine holes 221 even without an exhaust port, can maintain the pressure at the inside of the flow path 210 at the pressure of the atmosphere as the outside of the flow path, and can suppress an increase in pressure at the inside of the flow path 210. Thus, even when the force caused by the affinity is a relatively weak force, the flow path 210 easily feeds the liquid. In addition, since the flow path 210 can feed the liquid with a force caused by the affinity, it is not necessary to pressurize the flow path 210 to feed the liquid. In addition, since the porous member does not exist in the portion through which the liquid passes, the flow path 210 can prevent the liquid particles from entering the fine hole of the porous member, and easily feeds the liquid. In addition, even when the viscosity of the liquid particles is high or the liquid is a biomaterial of protein, it is possible to prevent the liquid from being adsorbed to the porous member in the middle of the portion through which the liquid passes, closing the porous hole, and causing it difficult to feed the liquid. As described above, the flow path 210 can easily feed the liquid in the flow path 210.

The flow path 210 may be provided with the space 242 defined at one end of the flow path 210 and the space 243 defined at the other end of the flow path 210. The liquid may be introduced into the space 242 and fed from the space 242 to the space 243. The space 242 is an example of a first space. The space 243 is an example of a second space.

As a result, in the flow path 210, when the liquid is introduced into the space 242, the liquid can be fed to the space 243 by the affinity between the liquid and the second region. Therefore, the flow path 210 can assist in measuring the state of the liquid stored in the space 243 by the measurement device irradiating the space 243 with the measurement light.

The thickness of the space 243 and the opening area of the surface along the liquid feeding direction in the space 243 may be determined so as to define the space 243 suitable for measuring the liquid to be fed.

This allows, in the flow path 210, the liquid fed to the space 243 to stably scatter the measurement light and the state of the liquid can be stably measured based on the scattered light. For example, in the flow path 210, by making the space 243 thin, the connection path 245 of the spaces 242 and 243 defined in the same layer as the space 243 also becomes thin, the force caused by the affinity is easily transmitted to the liquid, and the liquid feeding speed can be increased. In addition, when the space 243 is excessively thin, the optical path length of the measurement light passing through the space 243 is shortened, and the measurement sensitivity decreases. Therefore, it is preferable that the thickness of the space 243 is a thickness suitable for measurement. In addition, for example, when the opening area of the space 243 is excessively small, the optical path length of the measurement light passing through the space 243 is shortened, and the measurement sensitivity decreases. In the flow path 210, by adjusting the opening area of the space 243 to an area suitable for measurement, the optical path length of the measurement light passing through the space 243 can be appropriately secured and the measurement sensitivity can be improved. Thus, it is preferable that the opening area of the space 243 is an area suitable for measurement. As described above, in the flow path 210, by setting the thickness and the opening area of the space 243 to values suitable for measurement, it is possible to achieve both liquid feeding performance and measurement performance.

The flow path 210 may be provided with a separation membrane that separates the component to be managed from the liquid. The separation membrane may be disposed in the space 242.

This allows the flow path 210 to separate the liquid introduced into the space 242 by the separation membrane. Thus, the measurement device provided with the flow path 210 can measure the components of the liquid separated by the separation membrane using the measurement light. Thus, in the flow path 210, when the state of the liquid is measured with the measurement device, even if a liquid in which different components are mixed is measured, it is possible to avoid measurement in a state where different components are mixed, and to assist in improving measurement accuracy.

The fluid may include a biological fluid. The separation membrane may be the blood cell separation membrane 244 that separates the biological fluid into a blood cell component as the component to be managed and a non-blood cell component.

This allows the flow path 210 to separate the blood cell component and the non-blood cell component by using the blood cell separation membrane 244. Thus, the flow path 210 can, for example, allow blood cell components to stay on the blood cell separation membrane 244 and allow non-blood cell components to enter the connection path 245. Therefore, the flow path 210 can assist in preventing the drain effluent management system 5 from mixing and measuring blood cell components having properties different from those of non-blood cells in the measurement of the state of non-blood cells.

In this case, the drain effluent management system 5 can improve the measurement accuracy of non-blood cell components. Thus, the drain effluent management system 5 can objectively evaluate the state of non-blood cells contained in drain effluent while improving the measurement accuracy of non-blood cell components. In addition, since the drain effluent management system 5 can separate the blood cells and the non-blood cells by membrane separation, it is not necessary to use a centrifugal separator, and the size and cost of apparatus for separating the blood cells from the non-blood cells can be reduced.

At least one of the first region and the second region may have a light-transmitting property to transmit the measurement light used for measuring the liquid to be fed.

This allows, in the flow path 210, the measurement light progressed through the first region or the second region to reach the liquid to be measured. Thus, the flow path 210 can assist the measurement of the state of the fed liquid. That is, the flow path 210 can assist the optical evaluation of the liquid by the measurement device.

The flow path 210 may be provided with the porous sheet 220 forming the first region, the affinity sheet 230 forming the second region, and the adhesive layer 240 including an adhesive 241 disposed between the porous sheet 220 and the affinity sheet 230. Since the adhesive layer 240 has a second through-hole penetrating in the thickness direction in a shape corresponding to the flow path 210, the flow path 210 may be defined by the porous sheet 220, the affinity sheet 230, and the adhesive layer 240. The porous sheet 220 is an example of a first sheet. The affinity sheet 230 is an example of a second sheet.

This allows the flow path 210 to define a space as the second through-hole between the porous sheet 220 and the affinity sheet 230. This space may be, for example, the spaces 242 and 243 and the connection path 245. Thus, the flow path 210 is formed so as to be surrounded by the porous sheet 220 and the affinity sheet 230. The flow path 210 can be maintained at the atmospheric pressure by the fine holes 221 of the porous sheet 220, and a force caused by the affinity can be obtained by the affinity sheet 230. Therefore, the flow path 210 can feed the introduced liquid without requiring pressing or the like.

The porous sheet 220 may have a non-affinity for the liquid to be fed. The affinity sheet 230 may have an affinity for the liquid to be fed. The adhesive layer 240 may have a non-affinity for the liquid to be fed.

This allows the flow path 210 to prevent the liquid from leaking outside via porous sheet 220, since the porous sheet 220 has a non-affinity for the liquid. In the flow path 210, since the affinity sheet 230 has an affinity for the liquid, even when the liquid particles have a high viscosity or the liquid is a protein biomaterial (for example, plasma), the liquid can be easily fed due to the affinity for the liquid. Since the adhesive layer 240 has a non-affinity for the liquid, the flow path 210 can prevent the liquid from leaking to the outside via the porous sheet 220. Therefore, for example, it is possible to prevent the liquid from leaking to the adhesive 241 of the adhesive layer 240, and to prevent the adhesive force of the adhesive 241 from weakening and to prevent separating (peeling) the porous sheet 220 and the affinity sheet 230.

The flow path 210 may be provided with the porous sheet 220 forming the first region and the affinity sheet 230 forming the second region. The porous sheet 220 and the affinity sheet 230 may be disposed to face each other. Since the porous sheet 220 or the affinity sheet 230 has a recessed portion recessed in the thickness direction in the shape corresponding to the flow path 210, the flow path 210 may be defined by the porous sheet 220 and the affinity sheet 230.

This allows the flow path 210 to define a space having a shape corresponding to the flow path 210 in the porous sheet 220 or the affinity sheet 230. This space may be, for example, the spaces 242 and 243 and the connection path 245. Therefore, the flow path 210 is formed so as to be surrounded by the porous sheet 220 and the affinity sheet 230. The flow path 210 can be maintained at the atmospheric pressure by the fine holes 221 of the porous sheet 220, and a force caused by the affinity can be obtained by the affinity sheet 230. Thus, the flow path 210 can feed the introduced liquid without requiring pressing or the like. In addition, the flow path 210 can reduce the thickness of the adhesive layer 240 by bonding the porous sheet 220 and the affinity sheet 230 without providing the adhesive layer 240.

The porous sheet 220 may have a non-affinity for the liquid to be fed. The affinity sheet 230 may have an affinity for the liquid to be fed.

This allows the flow path 210 to prevent the liquid from leaking outside via the porous sheet 220, since the porous sheet 220 has a non-affinity for the liquid. In the flow path 210, since the affinity sheet 230 has an affinity for the liquid, even when the liquid particles have a high viscosity or the liquid is a protein biomaterial (for example, plasma), the liquid can be easily fed due to the affinity for the liquid.

The porous sheet 220 and the affinity sheet 230 may have flexibility such that the porous sheet 220 and the affinity sheet 230 can be bent in the arrangement direction where the porous sheet 220 and the affinity sheet 230 are arranged.

This allows the flow path 210 to have flexibility and to be easily deformed. Thus, the flow path 210 can be used as the measurement tape 200 by being wound up, for example, in a circular shape.

The component to be managed may include an enzyme or bilirubin.

Accordingly, the flow path 210 can feed the enzyme or bilirubin. Thus, the flow path 210 can assist, for example, the drain effluent sensor 10 in managing the components of the enzyme or bilirubin discharged from or introduced by the patient and measuring the condition of the patient.

The liquid may be a drain effluent.

Accordingly, the flow path 210 can feed at least a portion of the components contained in the drain effluent sensor 10. Thus, the flow path 210 can assist, for example, the drain effluent sensor 10 in managing the drain effluent discharged from the patient and measuring the condition of the patient.

In addition, the measurement tape 200 is provided with the above-described flow path 210, and the porous sheet 220 forming the first region and the affinity sheet 230 forming the second region, which are included in the flow path 210, have flexibility such that the porous sheet 220 and the affinity sheet 230 can be bent in the arrangement direction where the porous sheet 220 and the affinity sheet 230 are arranged.

Accordingly, even when a portion through which the liquid passes has no a porous member and no capillary force generated in the porous member as a drive force for liquid feeding is caused, the measurement tape 200 can feed the liquid by the force caused by the affinity of the second region. In addition, the measurement tape 200 allows air to pass through the fine holes 221 even without an exhaust port, and can maintain the pressure at the inside of the flow path 210 at the pressure of the atmosphere as the outside of the flow path, and can suppress an increase in pressure at the inside of the flow path 210. Therefore, even when the force caused by the affinity is a relatively weak force, the measurement tape 200 easily feeds the liquid. In addition, since the measurement tape 200 can feed the liquid with a force caused by the affinity, it is not necessary to pressurize the flow path 210 to feed the liquid. In addition, since the portion through which the liquid passes has no porous member, the measurement tape 200 can prevent the liquid particles from entering the fine hole of the porous member, and easily feeds the liquid. In addition, even when the viscosity of the liquid particles is high or the liquid is a biomaterial of protein, the measurement tape 200 can prevent the liquid from being adsorbed to the porous member in the middle of the portion through which the liquid passes, closing the porous hole, and can prevent causing it difficult to feed the liquid. As described above, the measurement tape 200 can easily feed the liquid in the flow path 210.

In addition, by forming the tape shape like the measurement tape 200, the measurement tape 200 can repeatedly use the flow path 210 for feeding the liquid. In addition, by feeding out the portion of the flow path 210 that is used once and using the unused flow path 210, the measurement of a liquid (for example, biological fluid) can be performed cleanly, and this allows for suppressing a decrease in measurement accuracy caused by the liquid remaining for the previous measurement.

In addition, the measurement device measures using the measurement tape 200. The measurement device is provided with the measurement tape 200 having the flow path 210, the CPU 181, the LED 182, and the photo sensor 183. The flow path 210 has the space 242 defined at one end of the flow path 210 and the space 243 defined at the other end of the flow path 210. The liquid is introduced into the space 242 and is fed from the space 242 to the space 243. The LED 182 emits the space 243 with measurement light. The photo sensor 183 receives the scattered light in which the measurement light is scattered in the space 243. The CPU 181 measures the state of the liquid fed through the flow path 210 based on the scattered light. The measurement device is, for example, the drain effluent sensor 10. The CPU 181 is an example of a measurement unit. The LED 182 is an example of a light source. The photo sensor 183 is an example of a light receiving unit.

Accordingly, even when a portion through which the liquid passes has no porous member and no capillary force generated in the porous member as a drive force for liquid feeding is caused, the measurement device can feed the liquid by a force caused by the affinity of the second region. In addition, the measurement device can allow air to pass through the fine holes 221 even without an exhaust port, can maintain the pressure at the inside of the flow path 210 at the pressure of the atmosphere as the outside of the flow path, and can suppress an increase in pressure at the inside of the flow path 210. Therefore, even when the force caused by the affinity is a relatively weak force, the measurement device easily feeds the liquid. In addition, since the measurement device can feed the liquid with a force caused by the affinity, it is not necessary to pressurize the flow path 210 to feed the liquid. In addition, since the porous member does not exist in the portion through which the liquid passes, the measurement device can prevent the liquid particles from entering the fine hole of the porous member, and easily feeds the liquid. In addition, even when the viscosity of the liquid particles is high or the liquid is a biomaterial of protein, it is possible for the measurement device to prevent the liquid from being adsorbed to the porous member in the middle of the portion through which the liquid passes, closing the porous hole, and causing it difficult to feed the liquid. As described above, the measurement device can easily feed the liquid in the flow path 210.

In addition, the measurement device can feed the liquid introduced into the space 242 to the space 243 due to the affinity between the liquid and the second region. The measurement device can measure the state of the liquid stored in the space 243 by irradiating the space 243 with the measurement light. In this case, since the size of the space 243 is invariable, the amount of liquid stored in the space 243 is constant. A measurement result for a constant amount of liquid can be obtained every time, and the liquid can be stably measured.

In addition, the drain effluent management system 5 can extract the component to be managed that affects the drain effluent from the drain tube to the outside. In addition, the drain effluent management system 5 can measure the extracted component to be managed, and can derive measurement data based on the measurement result. In addition, the drain effluent management system 5 displays time-series data representing the change over time of the measurement data on the display 21. This allows for performing evaluation based on time-series data instead of visual evaluation (or in addition to visual evaluation) when the user (doctor and nurse, other medical staff) evaluates the state of the drain effluent. Therefore, the user can more objectively evaluate the patient's recovery tendency as compared with the evaluation based only on visual observation.

In addition, similarly to the drain effluent, the component to be managed affecting the biological fluid can be extracted from the drain tube 30 to the outside for the biological fluid other than the drain effluent. In addition, the extracted component to be managed can be measured, and measurement data based on the measurement result can be derived. In addition, the time-series data representing the change over time of the measurement data is displayed on the display 21. This allows for performing evaluation based on time-series data instead of visual evaluation (or in addition to visual evaluation) when the user evaluates the state of the biological fluid. Therefore, the user can more objectively evaluate the patient's recovery tendency based on the biological fluid as compared with the evaluation based only on visual observation.

In addition, the drain effluent management system 5 can measure, for example, the amount of an enzyme, the amount of bilirubin, or the amount of blood cells discharged from or introduced into the patient. This makes it easy to grasp the state of the patient.

In addition, the drain effluent management system 5 can easily separate the blood cells from the non-blood cells by utilizing a difference in adsorption force of the blood cell separation membrane 244. In addition, the drain effluent management system 5 can derive (for example, calculate) the concentration of the non-blood cells whose correspondence with, for example, the absorbance is uniquely determined based on the absorbance by measuring the absorbance of the non-blood cells.

In addition, the drain effluent management system 5 can distinguish and detect each state of the blood cells and the non-blood cells by irradiating each of the region where the components of blood cells stay (for example, space 242 and blood cell separation membrane 244) and the region where the components of non-blood cells stay (for example, space 243) with the measurement light.

In addition, the drain effluent management system 5 can color and measure the enzyme, even when the colorless enzyme is the component to be managed, by allowing the enzyme separated by the blood cell separation membrane 244 to react with the reagent 231 and measuring the absorbance of the reacted enzyme. Therefore, when the colored enzyme is irradiated with the measurement light, the enzyme can scatter the measurement light, and the photo sensor 183 can receive the scattered light. Thus, even when the colorless enzyme is the component to be managed, the drain effluent management system 5 can measure the absorbance of the enzyme and can derive the concentration of the enzyme based on the absorbance.

In addition, the blood cell separation membrane 244 may be a glass membrane made by bundling glass fibers to be formed into a sheet shape. In this case, the drain effluent management system 5 can prevent the non-blood cells from being insufficiently acquired due to the small holes of the monolith membrane as compared with the monolith membrane by using a glass membrane as the blood cell separation membrane 244. The amount of non-blood cells necessary and sufficient for measurement can be ensured. In addition, by using the glass membrane as the blood cell separation membrane 244, since there is no holes unlike the cellulose membrane, the drain effluent management system 5 can prevent from passing through both blood cells and non-blood cells. The blood cells and the non-blood cells can be suitably separated by the capillary action of glass fibers. In addition, the drain effluent management system 5 can easily handle the blood cell separation membrane 244 similarly to the nonwoven fabric by using the blood cell separation membrane 244 made by bundling glass fibers to be formed into a sheet shape.

In addition, the drain effluent management system 5 has the effluent sampling mechanism 110 that can sample drain effluent with a simple configuration, is good in portability, and is easy to carry. This eliminates the need for the patient's staying at the fixed position at the time of sampling, and improves the flexibility of the user at the time of sampling. In addition, the drain effluent management system 5 can stabilize the amount of drain effluent present between the two points by keeping the distance between the two points in the main tube 130 unchanged during measurement. The amount of the drain effluent is one sampling amount. In addition, the drain effluent management system 5 can completely drain the drain effluent of one sampling amount by one pressing by allowing the drain effluent to pass when pressed by the first pressing member 115 and not allowing the drain effluent to pass when not pressed by the first pressing member 115. This can prevent the liquid from remaining in the sub-tube 133. Therefore, the drain effluent management system 5 can prevent the drain effluent from being mixed with the previous drain effluent in each sampling, and can derive measurement data with high measurement accuracy according to the sampling timing.

This application is based on Japanese patent application filed on Jan. 19, 2018 (Japanese Patent Application No. 2018-007385), the contents of which are incorporated herein by reference.

### Industrial Applicability

The present invention is useful for a flow path, a measurement tape, a measurement device, and the like that can easily feed the liquid in the flow path.

### Reference Signs List

- 5: drain effluent management system
- 10: drain effluent sensor
- 10A: TCI pump
- 10z: case
- 10y: through-hole
- 20: drain effluent monitor
- 20z: case
- 21: display
- 22: graph
- 23: explanatory text
- 24: meter
- 25: status marker
- 26: CPU
- 27: memory
- 28: wireless chip
- 30: drain tube
- 30A: medication tube
- 40: drain bag
- 41: inflow tube
- 110: effluent sampling mechanism
- 113,114: restriction member
- 113A, 114A, 115A: pressurizing unit
- 113B, 114B: partition plate
- 115: first pressing member
- 115B: pressing plate
- 130: main tube
- 130c: tube central portion
- 133: sub-tube
- 133z: flow path
- 150: blood cell separation and enzyme reaction mechanism
- 171: feed reel
- 172: winding reel
- 175: motor
- 176,177: roller
- 180: sensor unit
- 180z: case
- 181: CPU
- 182,182A, 182B: LED
- 183: photo sensor
- 184: wireless chip
- 185: battery
- 186: pressurizing unit drive unit
- 187: motor drive unit
- 188: circuit substrate
- 200: measurement tape
- 210: flow path
- 220: porous sheet
- 221: fine hole
- 222: through-hole
- 230: affinity sheet
- 231: reagent
- 240: adhesive layer
- 241: adhesive
- 242,243: space
- 244: blood cell separation membrane
- 245: connection path
- L1, L2: broken line
- Lq: drain effluent
- PA1: patient
- sq, sq1, sq2: sampling liquid

## Claims

1. A flow path for feeding a liquid,
wherein at least a portion of the flow path comprises:
a first region having a plurality of first through-holes for maintaining a pressure in the flow path at atmospheric pressure; and
a second region having an affinity for the liquid, and
wherein the liquid is fed by a force caused by the affinity for the second region.

2. The flow path according to claim 1, further comprising:
a first space defined at one end of the flow path; and
a second space defined at the other end of the flow path,
wherein the liquid is introduced into the first space, and is fed from the first space to the second space.

3. The flow path according to claim 2,
wherein a thickness of the second space and an opening area of a surface along a liquid feeding direction in the second space are determined so as to define the second space suitable for measuring the liquid to be fed.

4. The flow path according to claim 2 or 3, further comprising:
a separation membrane that separates a component to be managed from the liquid,
wherein the separation membrane is disposed in the first space.

5. The flow path according to claim 4,
wherein the liquid includes a biological fluid, and the separation membrane is a blood cell separation membrane that separates the biological fluid into a blood cell component as the component to be managed and a non-blood cell component.

6. The flow path according to any one of claims 1 to 5, wherein
wherein at least one of the first region and the second region has a light-transmitting property to transmit measurement light used for measuring the liquid to be fed.

7. The flow path according to any one of claims 1 to 6, further comprising:
a first sheet forming the first region;
a second sheet forming the second region; and
an adhesive layer including an adhesive disposed between the first sheet and the second sheet,
wherein the adhesive layer has a through-hole penetrating in a thickness direction in a shape corresponding to the flow path, so that the flow path is defined by the first sheet, the second sheet, and the adhesive layer.

8. The flow path according to claim 7, wherein
wherein the first sheet has a non-affinity for the liquid to be fed, the second sheet has an affinity for the liquid to be fed, and the adhesive layer has a non-affinity for the liquid to be fed.

9. The flow path according to any one of claims 1 to 8, further comprising:
a first sheet forming the first region; and
a second sheet forming the second region,
wherein the first sheet and the second sheet are disposed to face each other, and
the first sheet or the second sheet has a recessed portion recessed in the thickness direction in the shape corresponding to the flow path, so that the flow path is defined by the first sheet and the second sheet.

10. The flow path according to claim 9,
wherein the first sheet has a non-affinity for the liquid to be fed, and the second sheet has an affinity for the liquid to be fed.

11. The flow path according to any one of claims 7 to 10,
wherein the first sheet and the second sheet have flexibility to be bendable in an arrangement direction where the first sheet and the second sheet are arranged.

12. The flow path according to claim 4 or 5,
wherein the component to be managed includes an enzyme or bilirubin.

13. The flow path according to any one of claims 1 to 12,
wherein the liquid is a drain effluent.

14. A measurement tape comprising the flow path according to claim 1,
wherein a first sheet forming the first region and a second sheet forming the second region, which are included in the flow path, have flexibility to be bendable in an arrangement direction where the first sheet and the second sheet are arranged.

15. A measurement device which performs measurement using the measurement tape according to claim 14, the device comprising:
the measurement tape including the flow path;
a light source;
a light receiving unit; and
a measurement unit,
wherein the flow path includes a first space defined at one end of the flow path and a second space defined at the other end of the flow path,
the liquid is introduced into the first space, and is fed from the first space to the second space,
the light source emits measurement light to the second space,
the light receiving unit receives scattered light in which the measurement light is scattered in the second space, and
the measurement unit measures a state of the liquid fed through the flow path based on the scattered light.
